# EUROPÄISCHE PATENTSCHRIFT

(11) **EP 3 766 437 B1**
(45) Veröffentlichungstag und Bekanntmachung des Hinweises auf die Patenterteilung: **10.05.2023**
(21) Anmeldenummer: 20178889.0
(22) Anmeldetag: 09.06.2020
(51) Int. Cl.: A61B 17/14, A61B 17/16

(54) **KRAFTMASCHINE**
POWER MACHINE
MACHINE DE PUISSANCE

(30) Priorität: 18.07.2019 DE 102019004960
(43) Veröffentlichungstag der Anmeldung: 20.01.2021
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- DE-A1- 2 238 063
- DE-A1-102009 022 692
- DE-B4-102009 022 692
- DE-C2- 2 238 063
- US-A- 5 554 011
- US-B1- 6 383 203

## Beschreibung

Die Erfindung betrifft eine Kraftmaschine umfassend ein Arbeitssystem, aufweisend ein erstes Kolben-Zylinder-System, umfassend einen Arbeitskolben und einen Arbeitszylinder, wobei der Arbeitskolben den Arbeitszylinder in einen ersten Arbeitszylinderabschnitt und einen zweiten Arbeitszylinderabschnitt unterteilt, ein Ventilsystem, aufweisend einen ersten Ventilanschluss und ein Ventilelement, wobei das Ventilsystem und das Arbeitssystem gasleitend verbunden sind, der erste Ventilanschluss mit einer Unterdruckquelle verbindbar ist und das Ventilelement derart beweglich in dem Ventilsystem angeordnet ist, dass das Ventilelement in einer ersten Ventilposition den ersten Ventilanschluss mit dem ersten Arbeitszylinderabschnitt und in einer zweiten Ventilposition den ersten Ventilanschluss mit dem zweiten Arbeitszylinderabschnitt gasleitend verbindet. Die Erfindung betrifft weiterhin eine medizinische Vorrichtung aufweisend die erfindungsgemäße Kraftmaschine.

### Hintergrund der Erfindung

Im Umfeld chirurgischer Operationen werden medizinische Vorrichtungen zum Sägen, Fräsen, Bürsten, Bohren oder Besprühen von Knochenmaterial in breitem Umfang im Bereich septischer Revisionen eingesetzt. Dabei wird infiziertes Gewebe durch die medizinischen Vorrichtungen behandelt oder entfernt. Gegenwärtig kommen in solchen medizinischen Vorrichtungen meist elektrisch oder mit Druckluft betriebene Kraftmaschinen zum Betreiben der medizinischen Vorrichtungen zum Einsatz, wobei beide Antriebsquellen ihre jeweiligen Nachteile mit sich bringen.

Beispielsweise führt der Einsatz von Elektromotoren zu einer nicht unerheblichen Gewichtszunahme der medizinischen Vorrichtung, welche dem Operator die Handhabung erschwert. Zudem erhöht ein Elektromotor die Kosten der medizinischen Vorrichtung durch den Elektromotor selbst, als auch durch etwaig notwendige externe Stromquellen oder Batterien. Durch die hohen Beschaffungskosten eines Elektromotors ist eine Einmalnutzung der medizinischen Vorrichtung nicht wirtschaftlich, wodurch die Betriebskosten der medizinischen Vorrichtung durch eine für die Mehrfachnutzung notwendige Sterilisation zusätzlich erhöht sind.

Auf Druckluft basierende Antriebsvorrichtungen enthalten meist Lamellenmotoren. Problematisch gestaltet sich dabei, dass nicht sterile Druckluft zum Betreiben der Kraftmaschine verwendet wird, welche anschließend, aufgrund Kontaminationsgefahr, durch ein Schlauchsystem aus dem Operationssaal abgeführt werden muss. Der so erhöhte Aufwand schlägt sich in gesteigerten Kosten nieder.

Daher werden Bemühungen unternommen, durch Unterdruck betriebene Kraftmaschinen bereitzustellen, mittels derer medizinische Vorrichtungen einfach, sicher und kostengünstig betrieben werden können. Derartige Kraftmaschinen sind beispielsweise in der US 9,861,770 B1 und der US 5,554,011 A1 beschrieben.

In der US 8,292,909 B1 wird eine durch Unterdruck betriebene, doppeltwirkende Kraftmaschine zum Antreiben einer medizinischen Vorrichtung beschrieben. Die abwechselnde Beaufschlagung des Arbeitskolbens mit Unterdruck, und damit die Funktionsweise der Kraftmaschine, wird dabei durch die Auslenkung des Arbeitskolbens selbst gesteuert. Nachteilig ist, dass eine unzureichende Auslenkung des Arbeitskolbens zu einem Ausbleiben weiterer Schaltvorgänge führt, was eine erhöhte Verklemmungsgefahr, beispielsweise bei Sägevorgängen, zur Folge hat. Zudem beeinflusst die Arbeitslast in direkter Weise die Arbeitsfrequenz der Kraftmaschine, was eine Handhabung der medizinischen Vorrichtung durch den Anwender, beispielsweise durch ruckartige Bewegungen der medizinischen Vorrichtung, erschwert und zu ungleichmäßigen Arbeitsergebnissen, wie beispielsweise unsauberen Sägeabschnitten, führt.

In der DE 10 2009 022692 A1 wird eine Vorrichtung zum Applizieren eines medizinisch verriegelbaren Clips in einen Gewebebereich beschreiben, wobei die Vorrichtung ein Arbeitssystem, ein Ventilsystem und ein Steuersystem zur Steuerung des Ventilsystems umfasst.

In der DE 22 38 063 A1 wird ein strömungsmittelbetätigtes Instrument zum Greifen, Schneiden und Durchbohren von Knochen, Gewebe und anderen Strukturen beschrieben, wobei das Instrument, wobei das Instrument ein Arbeitssystem, ein Ventilsystem und ein Steuersystem umfasst.

In der US 5,554,011 A wird eine vakuumbetriebene Pumpe umfassend einen federbetriebenen Kolben beschrieben, wobei der Kolben unter Einfluss von Vakuum gegen die Rückstellkraft der Feder bewegt wird und wobei ein verzögert wirkendes Ventil ist mit einer Vakuumkammer gekoppelt ist, um den Eintritt von Umgebungsluft in die Kammer zu ermöglichen, nachdem nachdem der Kolben gegen die Rückstellkraft der Feder bewegt wurde.

In der US 6,383,203 B1 wird eine chirurgische Vorrichtung zur Entfernung eines Glaskörpers umfassend ein Arbeitssystem, ein Ventilsystem und ein Steuersystem zur Steuerung des Ventilsystems beschrieben.

### Aufgaben

Eine Aufgabe der vorliegenden Erfindung ist es, einen oder mehrere der sich aus dem Stand der Technik ergebenden Nachteile zumindest teilweise zu überwinden.

Im Speziellen basiert die Erfindung auf dem Ziel, Kraftmaschinen bereitzustellen, welche durch Unterdruckquellen angetrieben werden können. Die Kraftmaschine soll mit gleichbleibendem Arbeitsergebnis, zuverlässig und sicher, auch unter hoher Arbeitsbelastung, betrieben werden können. Die Kraftmaschine soll, unabhängig von der Arbeitsbelastung, mit einer konstanten Arbeitsfrequenz betrieben werden können. Das Ziel ist zudem, die anfallenden Kosten für die Kraftmaschine selbst, als auch deren Betrieb, zu minimieren, so dass eine Einmalnutzung der Kraftmaschine aus wirtschaftlichen Gründen möglich ist. Die Kraftmaschine soll im Wesentlichen aus Kunststoff gebildet sein oder vollständig aus Kunststoff bestehen.

Eine weitere Aufgabe der Erfindung ist es, eine medizinische Vorrichtung bereitzustellen, mittels derer mindestens ein Teil der bereits beschriebenen Aufgaben zumindest zum Teil gelöst ist.

### Bevorzugte Ausführungsformen der Erfindung

Ein Beitrag zur mindestens teilweisen Erfüllung mindestens einer der zuvor genannten Aufgaben wird durch die Merkmale des unabhängigen Anspruchs geleistet. Die abhängigen Ansprüche stellen bevorzugte Ausführungsformen bereit, die zur mindestens teilweisen Erfüllung mindestens einer der Aufgaben beitragen.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A, bedeuten entsprechend als Werte Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen parallele Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%.

### Ausführliche Beschreibung

Ein erster Gegenstand der Erfindung betrifft eine Kraftmaschine, wie beispielsweise einen Motor, umfassend ein Arbeitssystem, aufweisend ein erstes Kolben-Zylinder-System, umfassend einen Arbeitskolben und einen Arbeitszylinder, wobei der Arbeitskolben den Arbeitszylinder in einen ersten Arbeitszylinderabschnitt und einen zweiten Arbeitszylinderabschnitt unterteilt,
ein Ventilsystem, aufweisend einen ersten Ventilanschluss und ein Ventilelement, wobei das Ventilsystem und das Arbeitssystem gasleitend verbunden sind, der erste Ventilanschluss mit einer Unterdruckquelle verbindbar ist und das Ventilelement derart beweglich in dem Ventilsystem angeordnet ist, dass das Ventilelement in einer ersten Ventilposition den ersten Ventilanschluss mit dem ersten Arbeitszylinderabschnitt und in einer zweiten Ventilposition den ersten Ventilanschluss mit dem zweiten Arbeitszylinderabschnitt gasleitend verbindet,
ein Steuersystem, wobei das Steuersystem ein zweites Kolben-Zylinder-System, aufweisend einen Steuerkolben und einen Steuerzylinder, umfasst, wobei der Steuerkolben derart mit dem Ventilelement verbunden ist, dass eine zyklische Bewegung des Steuerkolbens im Steuerzylinder das Ventilelement zwischen der ersten Ventilposition und der zweiten Ventilposition bewegt,
wobei der erste Ventilanschluss über eine erste Ventilsystemzuleitung mit einem Ventilschalter verbunden ist, wobei der Ventilschalter die erste Ventilsystemzuleitung in einer ersten Ventilschalterposition reversibel gasleitend verschließt und in einer zweiten Ventilschalterposition die erste Ventilsystemzuleitung reversibel gasleitend öffnet,
**dadurch gekennzeichnet, dass**
zwischen Ventilsystem und Ventilschalter eine zweite Ventilsystemzuleitung und eine dritte Ventilsystemzuleitung angeordnet ist, wobei der Ventilschalter in der ersten Ventilschalterposition das Ventilsystem über die zweite Ventilsystemzuleitung mit der Unterdruckquelle gasleitend verbindet und in der zweiten Ventilschalterposition das Ventilsystem über die dritte Ventilsystemzuleitung mit der Umgebungsatmosphäre der Kraftmaschine gasleitend verbindet.

Die Kraftmaschine weist ein Arbeitssystem auf. Unter einem Arbeitssystem ist der Teil der Kraftmaschine zu verstehen, welcher eine von der Kraftmaschine genutzte Energie einer Energiequelle, beispielsweise Unterdruck, in außerhalb der Kraftmaschine nutzbare mechanische Energie umwandelt. Aufgrund der leichten Verfügbarkeit in Operationssälen und da eine Entsorgung kontaminierter Luft, wie sie bei der Verwendung von Überdruck anfallen würde, nicht notwendig ist, ist Unterdruck als Energiequelle bevorzugt. Dabei kann der Unterdruck beispielsweise durch eine speziell zum Betreiben der Kraftmaschine vorgesehene Vakuumpumpe oder durch eine in den meisten Operationssälen vorhandene Ringleitung für Unterdruck zur Verfügung gestellt werden.

Eine mit einer Unterdruckquelle betriebene Kraftmaschine kann auch als Vakuummotor bezeichnet werden.

Die Umwandlung des Unterdrucks in außerhalb der Kraftmaschine nutzbare mechanische Energie wird durch das erste Kolben-Zylinder-System des Arbeitssystems bewerkstelligt. Das erste Kolben-Zylinder-System weist hierfür den hohlen Arbeitszylinder mit dem im Arbeitszylinder reversibel axial beweglichen Arbeitskolben auf, wobei der Innenumfang des Arbeitszylinders und der Außenumfang des Arbeitskolbens einen um Wesentlichen gleichen Umfang besitzen. Der Arbeitskolben unterteilt den Arbeitszylinder in den ersten Arbeitszylinderabschnitt und den zweiten Arbeitszylinderabschnitt. Dabei verhindert der Arbeitskolben einen direkten Gasaustausch zwischen erstem Arbeitszylinderabschnitt und zweitem Arbeitszylinderabschnitt. Das erste Kolben-Zylinder-System ist derart ausgestaltet, dass beide Arbeitszylinderabschnitte sowohl mit der Unterdruckquelle, als auch mit der Umgebungsatmosphäre der Kraftmaschine verbunden oder verbindbar sind. Dies ermöglicht eine durch Unterdruck ausgelöste reversible axiale Bewegung des Arbeitskolbens innerhalb des Arbeitszylinders. In einer ersten Ausgestaltungsform weisen die Arbeitszylinderabschnitte jeweils einen separaten Anschluss für den Unterdruck und die Umgebungsatmosphäre auf. In einer weiteren, bevorzugten, Ausgestaltungsform weisen die Arbeitszylinderabschnitte jeweils nur einen Anschluss auf, welcher abwechselnd mit Unterdruck und mit der Umgebungsatmosphäre beaufschlagt werden kann. Beim Betrieb der Kraftmaschine ist entweder der erste Arbeitszylinderabschnitt oder der zweite Arbeitszylinderabschnitt mit der Unterdruckquelle und der jeweils andere Arbeitszylinderabschnitt mit der Umgebungsatmosphäre verbunden. Somit herrscht in einem der beiden Arbeitszylinderabschnitte im Vergleich zum anderen Arbeitszylinderabschnitt ein Unterdruck, was in einer axialen Bewegung des Arbeitskolbens resultiert. Die Bewegung des Arbeitskolbens verkleinert dabei den unter Unterdruck stehenden Arbeitszylinderabschnitt und vergrößert gleichzeitig den mit der Umgebungsatmosphäre verbundenen Arbeitszylinderabschnitt. Die Verbindung mit der Umgebungsatmosphäre erlaubt die axiale Bewegung des Arbeitskolbens innerhalb des Arbeitszylinders, indem durch Nachströmen von Gas der für die Bewegung notwendige Druckunterschied zwischen ersten Arbeitszylinderabschnitt und zweitem Arbeitszylinderabschnitt aufrechterhalten wird. Die Umgebungsatmosphäre stellt für die Kraftmaschine eine isobare Gasquelle dar. Alternativ zur Verbindung mit der Umgebungsatmosphäre, können die Arbeitszylinderabschnitte auch mit einer anderen Gasquelle verbunden oder verbindbar sein. Alternative Gasquellen sind beispielsweise Druckgasflaschen oder die in den meisten Operationssälen vorhandenen Ringleitungen, welche mit Luft oder einer anderen gasförmigen Substanz, wie beispielsweise Stickstoff, gefüllt sind.

Zum Betreiben der Kraftmaschine wird abwechselnd der erste Arbeitszylinderabschnitt und der zweite Arbeitszylinderabschnitt mit Unterdruck beaufschlagt, was in einer zyklischen Bewegung des Arbeitskolben innerhalb des Arbeitszylinders resultiert.

Die Beaufschlagung des ersten Arbeitszylinderabschnitt und des zweiten Arbeitszylinderabschnitt mit Unterdruck wird durch das Ventilsystem gesteuert. Das Ventilsystem weist hierfür den ersten Ventilanschluss, das Ventilelement und ein Ventilgehäuse, in welchem das Ventilelement reversibel axial beweglich angeordnet ist, auf, wobei das Ventilsystem und das Arbeitssystem gasleitend verbunden sind. Der erste Ventilanschluss ist dabei sowohl mit einer Unterdruckquelle, als auch mit dem ersten Arbeitszylinderabschnitt oder dem zweiten Arbeitszylinderabschnitt gasleitend verbunden oder verbindbar. Um den ersten Arbeitszylinderabschnitt mit Unterdruck zu beaufschlagen, ist der erste Arbeitszylinderabschnitt über den ersten Ventilanschluss gasleitend mit der Unterdruckquelle verbunden. Um den zweiten Arbeitszylinderabschnitt mit Unterdruck zu beaufschlagen, ist der zweite Arbeitszylinderabschnitt über den ersten Ventilanschluss gasleitend mit der Unterdruckquelle verbunden. Ob der erste Arbeitszylinderabschnitt oder der zweite Arbeitszylinderabschnitt über den ersten Ventilanschluss mit der Unterdruckquelle verbunden ist, wird durch das Ventilelement geregelt. Dies geschieht, indem das Ventilelement die Verbindung zwischen dem ersten Arbeitszylinderabschnitt oder dem zweiten Arbeitszylinderabschnitt und dem ersten Ventilanschluss reversibel gasleitend öffnet oder verschließt.

Hierzu ist das Ventilelement reversibel beweglich in dem Ventilsystem angeordnet. In der ersten Ventilposition regelt das Ventilelement, dass der erste Arbeitszylinderabschnitt gasleitend mit der Unterdruckquelle verbunden ist und dass der zweite Arbeitszylinderabschnitt nicht gasleitend mit der Unterdruckquelle verbunden ist. In der zweiten Ventilposition regelt das Ventilelement, dass der zweite Arbeitszylinderabschnitt gasleitend mit der Unterdruckquelle verbunden ist und dass der erste Arbeitszylinderabschnitt nicht gasleitend mit der Unterdruckquelle verbunden ist. Unter einer Ventilposition ist eine räumliche Anordnung des Ventilelement innerhalb des Ventilsystems, insbesondere innerhalb des Ventilzylinders, zu verstehen. Das Ventilelement kann die gasleitende Verbindung zwischen dem ersten Arbeitszylinderabschnitt oder dem zweiten Arbeitszylinderabschnitt und dem ersten Ventilanschluss auf unterschiedliche Arten öffnen und verschließen. In einer ersten Ausgestaltungsform ist das Ventilelement reversibel axial drehbar innerhalb des Ventilsystems angeordnet, so dass die Verbindung zwischen ersten Arbeitszylinderabschnitt oder zweiten Arbeitszylinderabschnitt und erstem Ventilanschluss durch eine Drehung des Ventilelements in die erste Ventilposition oder die zweite Ventilposition gasleitend geöffnet oder verschlossen wird. In einer weiteren, bevorzugten, Ausgestaltungsform, ist das Ventilelement reversibel axial beweglich im Ventilsystem angeordnet. Hierbei bewirkt die axiale Bewegung in die erste Ventilposition und die zweite Ventilposition, dass die Verbindung zwischen ersten Arbeitszylinderabschnitt oder zweitem Arbeitszylinderabschnitt und erstem Ventilanschluss gasleitend geöffnet oder verschlossen wird.

In der Ausgestaltungsform mit dem innerhalb des Ventilsystems reversibel axial beweglich angeordnetem Ventilelement, können das Ventilsystem und das Ventilelement auf unterschiedliche Weise zusammenwirken, um die abwechselnde Beaufschlagung der Arbeitszylinderabschnitte mit Unterdruck zu bewirken. In einer ersten Ausgestaltungsform weist das Ventilelement dafür rohrartige Durchführungen auf, welche, je nach Ventilposition, entweder die Unterdruckquelle oder eine Gasquelle mit dem ersten Arbeitszylinderabschnitt oder dem zweiten Arbeitszylinderabschnitt verbindet.

In einer weiteren, bevorzugten, Ausgestaltungsform ist das Ventilsystem als ein 5/2-Wegeventil ausgestaltet. Ein Wegeventil dient in der Fluidtechnik, beispielsweise der Pneumatik oder der Hydraulik, dazu, einen Weg für das Arbeitsmedium, wie beispielsweise Unterdruck, Überdruck oder Hydraulikflüssigkeit, zu öffnen oder zu verschließen. Wegeventile werden nach der Anzahl der Ventilanschlüsse und der Anzahl der Schaltstellungen dieser Ventilanschlüsse benannt. Ein 5/2-Wegeventil besitzt fünf Ventilanschlüsse mit jeweils zwei Schaltstellungen. In einer Schaltstellung ist der entsprechende Ventilanschluss für das Arbeitsmedium geöffnet, in der anderen Schaltstellung ist der entsprechende Ventilanschluss für das Arbeitsmedium verschlossen. Dazu weist das Ventilsystem neben dem ersten Ventilanschluss noch vier weitere Ventilanschlüsse auf. Dabei ist ein zweiter Ventilanschluss gasleitend mit dem zweiten Arbeitszylinderabschnitt und ein vierter Ventilanschluss gasleitend mit dem ersten Arbeitszylinderabschnitt verbunden. Der dritte Ventilanschluss und der fünfte Ventilanschluss verbinden das Ventilsystem mit der Gasquelle, vorzugsweise sind der dritte Ventilanschluss und der fünfte Ventilanschluss als Öffnungen des Ventilsystems zu der Umgebungsatmosphäre der Kraftmaschine ausgestaltet. Die Ventilanschlüsse sind dabei derart in dem Ventilsystem angeordnet, dass in axialer Ausrichtung des Ventilgehäuses immer nur ein Ventilanschluss vorhanden ist. In einer Ausgestaltungsform des 5/2-Wegeventils weist das Ventilelement einen zylinderartigen Ventilgrundkörper mit mindestens vier, um den Ventilgrundkörper radial umlaufenden, Ventilstegen und mindestens drei, jeweils räumlich zwischen den Ventilstegen angeordneten und um den Grundkörper radial umlaufenden, Ventilnuten auf. Das Ventilgehäuse weist einen zylinderartigen Ventilhohlraum auf, wobei der Außendurchmesser der Ventilstege im Wesentlichen gleich dem Innendurchmesser des Ventilhohlraums ist. Der Außendurchmesser der Ventilnuten ist kleiner als der Innendurchmesser des Ventilhohlraums. Die Ventilanschlüsse sind gasleitend mit dem Ventilhohlraum verbunden oder verbindbar. Um die Beaufschlagung des ersten Arbeitszylinderabschnitts oder des zweiten Arbeitszylinderabschnitts mit Unterdruck zu steuern, besitzen die Ventilnuten eine derartige axiale Ausdehnung, dass maximal zwei der Ventilanschlüsse über den Ventilhohlraum und die Ventilnuten verbunden oder verbindbar sind.

In der ersten Ventilposition ist der zweite Ventilanschluss gasleitend über eine Ventilnut mit dem dritten Ventilanschluss verbunden. Dies resultiert in einer gasleitenden Verbindung zwischen Gasquelle und zweitem Arbeitszylinderabschnitt. Des Weiteren ist in der ersten Ventilposition der erste Ventilanschluss über eine Ventilnut gasleitend mit dem vierten Ventilanschluss verbunden, was, bei mit dem ersten Ventilanschluss gasleitend verbundener Unterdruckquelle, zu einer Beaufschlagung des ersten Arbeitszylinderabschnitts mit Unterdruck führt.

In der zweiten Ventilposition ist hingegen der erste Ventilanschluss über eine Ventilnut gasleitend mit dem zweiten Ventilanschluss verbunden. Dies resultiert, bei gasleitender Verbindung zwischen Unterdruckquelle und ersten Ventilanschluss, zu einer Beaufschlagung des zweiten Arbeitszylinderabschnitts mit Unterdruck. Des Weiteren ist in der zweiten Ventilposition der vierte Ventilanschluss gasleitend über eine Ventilnut mit dem fünften Ventilanschluss verbunden. Dies resultiert in einer gasleitenden Verbindung zwischen Gasquelle und erstem Arbeitszylinderabschnitt.

Das Verbringen des Ventilelements in die erste Ventilposition und die zweite Ventilposition wird über das Steuersystem gesteuert. Dazu weist das Steuersystem das zweite Kolben-Zylinder-System auf. Das zweite Kolben-Zylinder-System umfasst den hohlen Steuerzylinder und den im Steuerzylinder reversibel axial beweglichen Steuerkolben, wobei der Innenumfang des Steuerzylinders und der Außenumfang des Steuerkolbens einen um Wesentlichen gleichen Umfang besitzen.

Der Steuerkolben ist derart mit dem Ventilelement gekoppelt, dass die zyklische Bewegung des Steuerkolbens innerhalb des Steuerzylinders das Ventilelement zyklisch zwischen der ersten Ventilposition und der zweiten Ventilposition bewegt. Die Kopplung zwischen Steuerkolben und Ventilelement kann auf unterschiedliche Arten verwirklicht sein.

In einer ersten Ausgestaltungsform besteht zwischen Steuerkolben und Ventilelement eine pneumatische Kopplung, derart, dass das Ventilelement durch Gasdruck, ausgelöst durch die Bewegung des Steuerkolbens und einem im Steuerzylinder befindlichem Gas, reversibel zwischen erster Ventilposition und zweiter Ventilposition verschoben wird.

In einer weiteren Ausgestaltungsform besteht zwischen Steuerkolben und Ventilelement eine hydraulische Kopplung, derart, dass das Ventilelement durch den Druck einer Flüssigkeit, ausgelöst durch die Bewegung des Steuerkolbens und der Flüssigkeit innerhalb des Steuerzylinders, reversibel zwischen erster Ventilposition und zweiter Ventilposition bewegt wird.

In einer weiteren, bevorzugten, Ausgestaltungsform sind der Steuerkolben und das Ventilelement mechanisch gekoppelt. Eine mechanische Kopplung ist bevorzugt, da eine mechanische Kopplung mit vergleichsweise wenigen Bauteilen baulich stabil verwirklicht werden kann. Dies senkt nicht nur das Risiko von Fehlfunktionen der Kraftmaschine, sondern kann auch kostengünstig umgesetzt werden.

Die mechanische Kopplung kann auf unterschiedliche Weisen verwirklicht werden. In einer ersten Ausgestaltungsform besteht eine indirekte mechanische Verbindung zwischen Steuerkolben und Ventilelement. Beispielsweise können Steuerkolben und Ventilelement durch einen Mitnehmer mechanisch gekoppelt sein. Der Mitnehmer überträgt dabei zeitversetzt die Bewegung des Steuerkolbens auf das Ventilelement. Beispielsweise führt eine Bewegung des Steuerkolbens zu einer zeitversetzen Bewegung des Ventilelements in die gleiche Richtung. Wechselt der Steuerkolben seine Bewegungsrichtung, führt das Ventilelement die ursprüngliche Bewegung aufgrund der Masseträgheit erst einmal fort, bevor der Mitnehmer dem Ventilelement zeitversetzt einen Impuls in die nun vollführte Bewegungsrichtung des Steuerkolbens vermittelt.

Steuerkolben und Ventilelement sind derart über den Mitnehmer gekoppelt, dass der Mitnehmer einen Bewegungsimpuls, insbesondere einen zeitversetzten Bewegungsimpuls, von Steuerkolben auf Ventilelement oder von Ventilelement auf Steuerkolben überträgt.

In einer weiteren, bevorzugten, Ausgestaltungsform der mechanischen Kopplung sind der Steuerkolben und Ventilelement direkt miteinander verbunden. Eine direkte mechanische Kopplung ist beispielsweise gegeben, falls Steuerkolben und Ventilelement einstückig ausgeformt sind. In einer Ausgestaltungsform sind Steuerkolben und Ventilelement form- und/oder kraftschlüssig oder über eine Stange miteinander verbunden. In einer weiteren Ausgestaltungsform sind Steuerkolben und Ventilelement stoffschlüssig, beispielsweise über eine Klebeverbindung, miteinander verbunden.

Eine weitere Ausgestaltungsform ist dadurch gekennzeichnet, dass der Steuerkolben und das Ventilelement einteilig ausgestaltet sind. Aufgrund der einfachen Herstellung, der direkten und sicheren Bewegungsübertragung und der geringen Anfälligkeit für Funktionsstörungen ist die einteilige Ausgestaltungsform von Steuerkolben und Ventilelement bevorzugt.

Eine direkte mechanische Kopplung von Steuerkolben und Ventilelement führt dazu, dass die räumliche Lage des Steuerkolbens einen direkten, zeitlich nicht verzögerten, Einfluss auf die räumliche Lage des Ventilelements besitzt. Eine Ausgestaltungsform der Kraftmaschine ist dadurch gekennzeichnet, dass eine Bewegung des Steuerkolbens in eine erste Steuerkolbenposition zu einer Bewegung des Ventilelements in die erste Ventilposition und eine Bewegung des Steuerkolbens in eine zweite Steuerkolbenposition zu einer Bewegung des Ventilelement in die zweite Ventilposition führt.

Die Bewegung des Steuerkolbens kann auf unterschiedliche Weisen ausgelöst werden. In einer ersten Ausgestaltungsform wird der Steuerkolben durch mechanische Einwirkungen in die erste Steuerkolbenposition und die zweite Steuerkolbenposition verbracht. Dies kann beispielsweise durch eine mechanische Kopplung mit einem Elektromotor geschehen.

Eine weitere Ausgestaltungsform der Kraftmaschine ist dadurch gekennzeichnet, dass das Steuersystem gasleitend mit dem Ventilsystem verbunden ist. Diese Ausgestaltungsform ist bevorzugt, da dadurch die zum Betreiben des Arbeitssystems verwendete Unterdruckquelle gleichzeitig auch zum Betreiben des Steuersystems eingesetzt werden kann und somit weitere, interne oder externe, Energiequellen nicht notwendig sind. Dies senkt zum einen die Komplexität der Kraftmaschine und damit die Anfälligkeit für Störungen, zum anderen werden dadurch sowohl die Kosten für die Kraftmaschine selbst, als auch die Kosten zum Betrieb der Kraftmaschine gesenkt.

Eine nach dem zitierten Stand der Technik aufgebaute Kraftmaschine kann eine mechanische Kopplung zwischen Arbeitssystem und Ventilsystem aufweisen, wobei eine Unterdruckquelle das Arbeitssystem und dieses wiederum durch eine Bewegung des Arbeitskolbens das Ventilsystem antriebt. Solch eine nach dem Stand der Technik operierende Kraftmaschine weist kein separates Steuersystem auf. Ein Vorteil eines separaten Steuersystems ist, dass dadurch die Bewegung des Ventilsystems, und damit die taktgebende Einheit für das Arbeitssystems, unabhängig von der Arbeitslast des Arbeitssystems betrieben wird. Beispielsweise hat das Sägen eines Knochens zwar einen Einfluss auf das Arbeitssystem, aber keinen Einfluss auf die Bewegung des Ventilsystem, wodurch bei einer Verklemmung der Säge in eine Richtung, die Kraftmaschine durch das Umschalten in eine entgegengesetzte Bewegungsrichtung des Arbeitssystems eine erhöhte Chance besitzt, die Säge ohne Zutun des Anwenders selbstständig zu befreien. Eine nach dem Stand der Technik aufgebaute Kraftmaschine wird bei einer verklemmten Säge solange in der Bewegungsrichtung des Arbeitssystems verbleiben, bis der Anwender der Kraftmaschine die Säge durch eigenes Zutun aus der Verklemmung befreit. Ein Umschalten der Bewegungsrichtung durch das Ventilsystems ist nicht möglich. Zudem hat ein separates Steuersystem den Vorteil, dass die Arbeitsfrequenz der Kraftmaschine unabhängig von der Arbeitslast des Arbeitssystems ist. Eine erhöhte Arbeitslast mag zwar einen Einfluss auf die Bewegungsgeschwindigkeit des Arbeitskolbens und/oder auf dessen Amplitude haben, aber nicht auf die Frequenz der Änderung der Bewegungsrichtung. Somit kommt es bei Verwendung der Kraftmaschine nicht zu unerwarteten Änderung der Arbeitsfrequenz, was zum einen die Handhabung der Kraftmaschine für den Anwender, als auch die Qualität des Arbeitsergebnisses verbessert. Beispielswiese wird durch eine gleichbleibende Arbeitsfrequenz das Sägeergebnis beim Sägen eines Knochens verbessert, da der Sägeschnitt gleichmäßiger und sauberer durchgeführt wird als bei wechselnder Arbeitsfrequenz.

Die gasleitende Verbindung zwischen Ventilsystem und Steuersystem, und damit der Betrieb des Steuersystems, kann auf verschiedene Weisen realisiert werden.

Eine Ausgestaltungsform der Kraftmaschine ist dadurch gekennzeichnet, dass der Steuerkolben den Steuerzylinder in einen ersten Steuerzylinderabschnitt und einen zweiten Steuerzylinderabschnitt unterteilt, wobei der erste Steuerzylinderabschnitt und der zweite Steuerzylinderabschnitt gasleitend mit dem Ventilsystem verbunden sind. Dadurch kann sowohl der erste Steuerzylinderabschnitt als auch der zweite Steuerzylinderabschnitt im Wechsel mit Unterdruck beaufschlagt werden. Dies führt zu einem zyklischen Verbringen des Steuerkolbens in die erste Steuerkolbenposition und die zweite Steuerkolbenposition.

Eine Ausgestaltungsform der Kraftmaschine ist dadurch gekennzeichnet, dass das Ventilelement in der ersten Ventilposition den ersten Ventilanschluss und den zweiten Steuerzylinderabschnitt und in der zweiten Ventilposition den ersten Ventilanschluss und den ersten Steuerzylinderanschluss gasleitend verbindet. Auf diese Weise wird, bei gasleitender Verbindung zwischen einer Unterdruckquelle und dem ersten Ventilanschluss, der erste Steuerzylinderabschnitt oder der zweite Steuerzylinderabschnitt mit Unterdruck beaufschlagt. Dies bedeutet, dass sowohl das Verbringen des Steuerkolbens in die erste Steuerkolbenposition, als auch das Verbringen des Steuerkolbens in die zweite Steuerkolbenposition durch Beaufschlagung des Steuerkolbens mit Unterdruck geschieht.

Das Ventilsystem kann auf unterschiedliche Arten konstruiert sein, um das Arbeitssystem, und optional auch das Steuersystem, zu betreiben.

Eine Ausgestaltungsform der Kraftmaschine ist dadurch gekennzeichnet, dass das Ventilsystem als 5/2-Wegeventil ausgestaltet ist.

Dazu weist das Ventilsystem neben dem ersten Ventilanschluss noch vier weitere Ventilanschlüsse auf. Dabei ist der zweite Ventilanschluss gasleitend mit dem zweiten Arbeitszylinderabschnitt und der vierte Ventilanschluss gasleitend mit dem ersten Arbeitszylinderabschnitt verbunden. Der dritte Ventilanschluss und der fünfte Ventilanschluss verbinden das Ventilsystem mit der Gasquelle, vorzugsweise sind der dritte Ventilanschluss und der fünfte Ventilanschluss als Öffnungen des Ventilsystems zur Umgebungsatmosphäre der Kraftmaschine ausgestaltet. Die Ventilanschlüsse sind dabei derart in dem Ventilsystem angeordnet, dass in axialer Erstreckung des Ventilgehäuses immer nur ein Ventilanschluss vorhanden ist. In einer Ausgestaltungsform des 5/2-Wegeventils weist das Ventilelement einen zylinderartigen Ventilgrundkörper mit mindestens vier, um den Ventilgrundkörper radial umlaufende, Ventilstege und mindestens drei, jeweils zwischen den Ventilstegen angeordneten und um den Grundkörper radial umlaufende, Ventilnuten auf. Das Ventilgehäuse weist einen zylinderartigen Ventilhohlraum auf, wobei der Außendurchmesser der Ventilstege im Wesentlichen gleich dem Innendurchmesser des Ventilhohlraums ist. Der Außendurchmesser der Ventilnuten ist kleiner als der Innendurchmesser des Ventilhohlraums. Dabei sind die Ventilanschlüsse gasleitend mit dem Ventilhohlraum verbunden. Um die Beaufschlagung des ersten Arbeitszylinderabschnitts oder des zweiten Arbeitszylinderabschnitts mit Unterdruck zu steuern, besitzen die Ventilnuten eine derartige axiale Ausdehnung in dem Ventilelement, dass maximal zwei der Ventilanschlüsse über den Ventilhohlraum und die Ventilnuten verbunden oder verbindbar sind.

Eine weitere Ausgestaltungsform der Kraftmaschine ist dadurch gekennzeichnet, dass der Steuerkolben den Steuerzylinder in einen ersten Steuerzylinderabschnitt und einen zweiten Steuerzylinderabschnitt unterteilt, wobei der erste Steuerzylinderabschnitt oder der zweite Steuerzylinderabschnitt gasleitend mit dem Ventilsystem verbunden ist. Somit wird nur das Verbringen des Steuerkolbens in die erste Steuerkolbenposition oder in die zweite Steuerkolbenposition mittels Druckdifferenz in den beiden Steuerzylinderabschnitten bewerkstelligt. Das Verbringen in die entsprechend andere Steuerkolbenposition muss daher auf andere Weise vonstattengehen, was auf unterschiedliche Weise bewirkt werden kann. Beispielsweise kann das Steuersystem eine mechanische Vorrichtung umfassen, welche den Steuerkolben, nachdem dieser mittels Unterdruck in die erste Steuerkolbenposition oder die zweite Steuerkolbenposition verbracht wurde, in die entsprechend andere Steuerkolbenposition verbringt. In einer Ausgestaltungsform ist die mechanische Vorrichtung entweder in dem Steuerzylinderabschnitt angebracht, welcher mit dem Steuersystem gasleitend verbunden ist, in dem Steuerzylinderabschnitt angebracht, welcher nicht gasleitend mit dem Steuerzylinder verbunden ist oder die mechanische Vorrichtung ist in beiden Steuerzylinderabschnitten angebracht. In einer weiteren Ausgestaltungsform ist die mechanische Vorrichtung außerhalb der Steuerzylinderabschnitte angebracht.

Eine Ausgestaltungsform der Kraftmaschine ist dadurch gekennzeichnet, dass der mit dem Ventilsystem verbundene Steuerzylinder ein Energiespeicherelement enthält. Das Energiespeicherelement dient dabei als mechanische Vorrichtung, welche den Steuerkolben, nach dem Verbringen in den mit Unterdruck beaufschlagten Steuerzylinderabschnitt, in Richtung des nicht gasleitend mit dem Ventilsystem verbundenen Steuerzylinderabschnitts verbringt. Das Energiespeichersystem kann unterschiedlich ausgeformt sein. Beispielsweise kann das Energiespeichersystem die für das Verschieben des Steuerkolbens benötigte Energie in Form von komprimierter Luft speichern oder in Gestalt einer Feder ausgeformt sein, wobei eine Feder aufgrund der einfachen Bauweise, der ausfallsicheren Funktionsweise und aus Kostengründen bevorzugt ist.

Die gasleitenden Verbindungen zwischen Ventilsystem und Arbeitssystem und optional zwischen Ventilsystem und Steuersystem können auf unterschiedliche Weise realisiert werden. Beispielsweise kann eine gasleitende Verbindung als rohrartige Verbindung, beispielsweise aus Metall oder Kunststoff, oder als schlauchartige Verbindung, beispielsweise aus Kunststoff, ausgestaltet sein. Schlauchartige Verbindungen aus Kunststoff sind aufgrund der flexibleren Bauweise, aufgrund des geringeren Gewichts und aus Kostengründen bevorzugt. Ein anderer Begriff für eine gasleitende Verbindung ist eine Zuleitung.

Die gasleitenden Verbindungen können unterschiedliche Längen und Querschnittsöffnungen aufweisen. Insbesondere die Querschnittsöffnungen sind bevorzugt so zu wählen, dass eine optimale Funktionsweise der Kraftmaschine sichergestellt ist. Da der Arbeitskolben normalerweise eine deutliche größere Arbeitslast als der Steuerkolben zu verrichten hat, ist es bevorzugt, dass der Arbeitskolben eine größere Querschnittsfläche als der Steuerkolben aufweist. Der Steuerkolben dient ausschließlich dem Verbringen des Ventilelements in die erste Ventilposition und zweite Ventilposition, wohingegen die Bewegung des Arbeitskolbens beispielsweise dazu genutzt wird, mittels eines Sägeblatts einen Knochen zu durchtrennen. Letzteres bedarf einer größeren Kraft und deswegen einer größeren Querschnittsfläche. Da die Querschnittsfläche einen direkten Einfluss auf das Volumen des umgebenden Zylinders hat, weist der Arbeitszylinder normalerweise ein größeres Volumen als der Steuerzylinder auf. Eine optimale Funktionsweise der Kraftmaschine liegt dann vor, wenn Arbeitskolben und Steuerkolben in gleicher Frequenz in den jeweiligen Zylindern bewegt werden. Da die Bewegung durch die Beaufschlagung der Kolben mit Unterdruck ausgelöst wird, sind die Querschnittsöffnungen der Zuleitungen bevorzugt derart aufeinander angepasst, dass trotz der unterschiedlich großen Volumina, der jeweiligen Arbeitszylinderabschnitt und Steuerzylinderabschnitt, gleich schnell evakuiert, also mit Unterdruck beaufschlagt, werden. Eine Ausgestaltungsform der Kraftmaschine ist dadurch gekennzeichnet, dass das Arbeitssystem über eine erste Arbeitssystemzuleitung und das Steuersystem über eine erste Steuersystemzuleitung gasleitend mit dem Ventilsystem verbunden ist, wobei der Quotient der Querschnittsöffnung der ersten Arbeitssystemzuleitung zur Querschnittsöffnung der ersten Steuersystemzuleitung gleich oder größer dem Quotienten der Summe der Volumina des ersten Arbeitszylinderabschnitts und des zweiten Arbeitszylinderabschnitts zur Summe der Volumina des ersten Steuerzylinderabschnitts und des zweiten Steuerzylinderabschnitts ist. So ist sichergestellt, dass die entsprechenden Volumina gleich schnell evakuiert werden und Arbeitszylinder und Steuerzylinder im Wesentlichen die gleiche Bewegungsfrequenz aufweisen.

Damit die Bewegung des Arbeitskolbens außerhalb der Kraftmaschine als mechanische Energie genutzt werden kann, ist der Arbeitssystem in einer Ausgestaltungsform der Kraftmaschine mit einem Bearbeitungselement, insbesondere zum Sägen, Fräsen, Bürsten, Bohren oder Besprühen von Knochenmaterial, verbunden. Dabei können Arbeitssystem und Bearbeitungsmittel auf unterschiedliche Weisen verbunden sein. Beispielsweise können Arbeitssystem und Bearbeitungsmittel direkt, ohne Verwendung weiterer Bauteile, miteinander verbunden sein.

Eine Ausgestaltungsform der Kraftmaschine ist dadurch gekennzeichnet, dass das Arbeitssystem ein Arbeitselement aufweist. Ein Arbeitselement ist ein Bauteil, welches eine Verbindung zwischen Arbeitssystem und Bearbeitungsmittel herstellt, derart, dass die Bewegungsenergie des Arbeitskolbens in eine außerhalb der Kraftmaschine nutzbare mechanische Energie umgewandelt werden kann. Dabei kann das Arbeitselement als eine pneumatische, hydraulische oder mechanische Verbindung zwischen Arbeitssystem und Bearbeitungsmittel ausgestaltet sein. Aufgrund der einfachen Konstruktionsweise, der geringen Anfälligkeit für Ausfälle und den vergleichsweise geringen Produktionskosten, ist eine mechanische Verbindung zwischen Arbeitssystem und Bearbeitungsmittel bevorzugt. Beispiele für als mechanische Verbindung ausgestaltete Arbeitselemente sind Stangen, Rohre und Pleuel.

Dabei kann das Arbeitselement auf unterschiedliche Weise mit dem Arbeitssystem verbunden sein. Aufgrund der einfachen Konstruktionsweise ist das Arbeitselement bevorzugt direkt, ohne Verwendung weiterer Bauteile, mit dem Arbeitskolben verbunden.

Eine Ausgestaltungsform der Kraftmaschine ist dadurch gekennzeichnet, dass das Arbeitselement kraft- und/oder formschlüssig mit dem Arbeitskolben verbunden ist oder dass das Arbeitselement und der Arbeitskolben einteilig ausgestaltet sind.

Eine Ausgestaltungsform der Kraftmaschine ist dadurch gekennzeichnet, dass das Arbeitselement mit einem Bearbeitungsmittel, insbesondere zum Sägen, Fräsen, Bürsten, Bohren oder Besprühen von Knochenmaterial, ausstattbar ist. Beispielsweise umfasst das Arbeitselement hierzu einen Schlitz, in den das Bearbeitungsmittel eingespannt werden kann, oder einen Bajonettanschluss, welcher mit einem entsprechenden Gegenstück am Bearbeitungsmittel zusammenwirken kann.

In einer Ausgestaltungsform der Kraftmaschine dient der erste Ventilanschluss dazu, die Kraftmaschine, insbesondere das Ventilsystem, gasleitend mit der Unterdruckquelle zu verbinden. Die gasleitende Zuleitung zwischen erstem Ventilanschluss und der Unterdruckquelle kann auf unterschiedliche Weisen ausgestaltet sein. In einer Ausgestaltungsform besteht zwischen erstem Ventilanschluss und Unterdruckquelle eine direkte gasleitenden Zuleitung, beispielsweise in Form eines Schlauches, so dass das Ventilsystem dauerhaft mit Unterdruck beaufschlagt ist, sofern die gasleitende Zuleitung nicht gelöst oder die Unterdruckquelle nicht abgeschaltet wird. In einer weiteren, bevorzugten, Ausgestaltungsform, ist der erste Ventilanschluss derart mit der Unterdruckquelle verbunden, dass die Zuleitung es gestattet, die Beaufschlagung der Kraftmaschine mit Unterdruck zu unterbrechen, ohne dass dafür die Zuleitung zwischen erstem Ventilanschluss und Unterdruckquelle gelöst oder die Unterdruckquelle abgeschaltet werden muss. Auf diese Weise kann der Anwender der Kraftmaschine, ohne großen Aufwand und ohne unnötige maschinelle Beanspruchung der Unterdruckquelle, nach Belieben in und außer Betrieb nehmen.

Die Kraftmaschine ist dadurch gekennzeichnet, dass der erste Ventilanschluss über eine erste Ventilsystemzuleitung mit einem Ventilschalter verbunden ist, wobei der Ventilschalter die erste Ventilsystemzuleitung ein einer ersten Ventilschalterposition reversibel gasleitend verschließt und in einer zweiten Ventilschalterposition die erste Ventilsystemzuleitung reversibel gasleitend öffnet.

Beispielsweise kann der Ventilschalter als Wegeventil ausgestaltet sein, durch welches der Anwender der Kraftmaschine mittels Betätigung eines Ventilschalterabzugs die erste Ventilsystemzuleitung reversibel gasleitend öffnet oder reversibel gasleitend verschließt um so die Kraftmaschine zu starten oder zu stoppen.

In einer Ausgestaltungsform der Kraftmaschine ist das Ventilelement derart in dem Ventilgehäuse angeordnet, dass das Verbringen des Ventilelements in die erste Ventilposition und/oder in die zweite Ventilposition, einen hohlraumartigen Ventilabschnitt an der Position innerhalb des Ventilgehäuses hinterlässt, von der das Ventilelement gerade fortbewegt wird. Da die fortscheitende Bewegung des Ventilelements das Volumen des Ventilabschnitts ständig vergrößert, muss, um das Entstehen eines ungewollten Unterdrucks innerhalb des Ventilabschnitts zu verhindern, ein Gaszustrom in den Ventilabschnitt erfolgen. Ein ungewollter Unterdruck innerhalb des Ventilabschnitts könnte sich nachteilig auf die Bewegung des Ventilelements auswirken. Beispielsweise könnte der ungewünschte Unterdruck dafür sorgen, dass das Ventilelement in einer Position innerhalb des Ventilgehäuses verharrt und der Betrieb der Kraftmaschine zum Erliegen kommt. Eine entsprechende Problematik ergibt sich bei der Bewegung des Ventilelements in Richtung des nun gasgefüllten Ventilabschnitts. Nun muss das Gas aus dem sich verkleinernden Ventilabschnitt entfernt werden, da sonst ein Überdruck entstünde, welcher wiederum negative Auswirkungen auf die Bewegung des Ventilelements ausüben würde. In einer Ausführungsform weist das Ventilgehäuse mindestens eine Ventilabschnittdurchführung zur Umgebungsatmosphäre auf, damit ein Luftzustrom in, als auch aus dem Ventilabschnitt stattfinden kann. In einer weiteren, bevorzugten, Ausführungsform weist das Ventilgehäuse nicht nur eine erste Ventilabschnittdurchführung zur Umgebungsatmosphäre auf, sondern auch eine zweite Ventilabschnittdurchführung, über welche der Ventilabschnitt mit der Unterdruckquelle verbindbar ist, wobei die Ventilabschnittdurchführungen derart ausgestaltet sind, dass der Ventilabschnitt entweder mit der Umgebungsatmosphäre oder mit der Unterdruckquelle verbunden ist.

Die Kraftmaschine ist dadurch gekennzeichnet, dass zwischen Ventilsystem und Ventilschalter eine zweite Ventilsystemzuleitung und eine dritte Ventilsystemzuleitung angeordnet ist, wobei der Ventilschalter in der ersten Ventilschalterposition das Ventilsystem über die zweite Ventilsystemzuleitung mit der Unterdruckquelle gasleitend verbindet und in der zweiten Ventilschalterposition das Ventilsystem über die dritte Ventilsystemzuleitung mit der Umgebungsatmosphäre der Kraftmaschine gasleitend verbindet.

Ein Vorteil dieser Ausgestaltungsform ist, dass das Ventilelement, und damit auch das Arbeitssystem, in der ersten Ventilschalterposition durch die Beaufschlagung des Ventilabschnitts mit Unterdruck immer in der gleichen, definierten, Stellung vorliegt. Die erste Ventilschalterposition stellt somit, bei angeschlossener Unterdruckquelle, einen definierten Ausgangszustand der Kraftmaschine dar. Dies erleichtert dem Anwender der Kraftmaschine das Anbringen der Bearbeitungsmittel an das Arbeitssystem, da der Arbeitskolben in einer definierten Position verbleibt, ohne verschiebbar zu sein. Erst das Umschalten des Ventilschalters in die zweite Ventilschalterposition verbindet den Ventilabschnitt mit der Umgebungsatmosphäre und erlaubt somit eine Bewegung des Ventilelement und damit auch eine Bewegung des Arbeitskolbens.

Die Kraftmaschine kann unterschiedliche Materialien umfassen oder daraus bestehen. Vorzugsweise sind die Materialien mechanische belastbar, beständig gegen Temperaturen von bis zu 50 °C und leicht zu sterilisieren.

Eine Ausgestaltungsform der Kraftmaschine ist dadurch gekennzeichnet, dass die Kraftmaschine aus Kunststoff, Metall oder aus einer Kombination von Kunststoff und Metall gefertigt ist.

Beispiele für Kunststoffe umfassen Polymethylmethacrylat, Polycarbonat, Polyoxymethylen und Polyamid. Beispiele für Metalle umfassen Aluminium, Eisen, Stahl, Edelstahl, Messing und Kupfer.

Eine Ausgestaltungsform der Kraftmaschine ist dadurch gekennzeichnet, dass der Arbeitskolben und der Steuerkolben derart gekoppelt sind, dass eine Steuerfrequenz des Steuerkolbens im Wesentlichen einflussfrei von der Arbeitsfrequenz des Arbeitskolbens ist.

Ein Vorteil dieser Ausgestaltungsform ist, dass das Steuersystem, während des Betriebs der Kraftmaschine, in immer der gleichen Steuerfrequenz betrieben werden kann und diese Steuerfrequenz in immer gleicher Weise auf das Arbeitssystem überträgt. Daher weist auch das Arbeitssystem immer die gleiche Arbeitsfrequenz auf. Eine hohe Arbeitsbelastung kann zwar die Bewegungsgeschwindigkeit und/oder die Amplitude der Bewegung des Arbeitskolbens beeinflussen, nicht aber die Frequenz. Dies sorgt für eine bessere Handhabbarkeit der Kraftmaschine, da der Anwender nicht mit Frequenzwechseln, und damit ruckartigen Ausschlägen der Kraftmaschine zurechtkommen muss. Zudem sorgt eine gleichbleibende Arbeitsfrequenz für gleichmäßigere Arbeitsergebnisse.

Je nach Einsatzgebiet der Kraftmaschine, kann der Arbeitskolbens unterschiedliche Durchmesser besitzen. Dabei weist der Arbeitskolben einen umso größeren Durchmesser auf, je größer die zu erwartende Arbeitsbelastung der Kraftmaschine ist.

Eine Ausgestaltungsform der Kraftmaschine ist dadurch gekennzeichnet, dass der Arbeitskolben einen Durchmesser zwischen 10 mm und 40 mm, bevorzugt zwischen 15 mm und 35 mm, noch bevorzugter zwischen 20 mm und 30 mm, aufweist.

Die beschriebene Kraftmaschine kann in unterschiedlichen Vorrichtungen Verwendung finden. Beispielsweise kann die Kraftmaschine in Sägen oder Bürsten zum Einsatz kommen. Aufgrund der einfachen Konstruktionsweise, der Möglichkeit, die Kraftmaschine aus günstigen Materialien, wie beispielsweise Kunststoffen, zu fertigen und der Ausnutzung von Unterdruck zum Betrieb der Kraftmaschine, welcher in Operationssälen normalerweise zur Verfügung steht, ist eine Verwendung in medizinischen Vorrichtungen bevorzugt. Vorzugsweise sind die medizinischen Vorrichtungen handgeführt, also mobil einsetzbar, und von einem Anwender allein zu bedienen.

Ein weiterer Gegenstand der Erfindung betrifft eine medizinische Vorrichtung, insbesondere zum Sägen, Fräsen, Bürsten, Bohren oder Besprühen von Knochenmaterial, dadurch gekennzeichnet, dass die medizinische Vorrichtung eine Kraftmaschine nach einer der vorhergehenden Ausführungsformen aufweist.

Die Kraftmaschine kann auf unterschiedliche Weise in der medizinischen Vorrichtung angeordnet sein. In einer Ausführungsform ist das Ventilsystem direkt angrenzend an das Arbeitssystem im Gehäuse der medizinischen Vorrichtung angeordnet. In einer weiteren, bevorzugten, Ausführungsform ist das Ventilsystem räumlich getrennt vom Arbeitssystem angeordnet, derart, dass das Gehäuse der medizinischen Vorrichtung möglichst kompakt und klein ausgestaltet sein kann.

Eine Ausgestaltungsform der medizinischen Vorrichtung ist dadurch gekennzeichnet, dass die medizinische Vorrichtung einen Griff aufweist, wobei das Ventilsystem innerhalb des Griffs angeordnet ist.

### Beispiele

Die Erfindung wird im Folgenden durch Beispiele weiter beispielhaft illustriert. Die Erfindung ist nicht auf die Beispiele beschränkt.

### Figuren

Es zeigen
- Fig. 1: eine schematische Zeichnung einer Kraftmaschine in einem Ausgangszustand,
- Fig. 2: die Kraftmaschine aus Fig. 1 in Betrieb in einer ersten Ventilposition,
- Fig. 3: die Kraftmaschine aus Fig. 1 und Fig. 2 in Betrieb in einer zweiten Ventilposition, und
- Fig. 4: ein Verfahren zum Behandeln eines Säugetiers mittels einer medizinischen Vorrichtung.

### Beschreibung der Figuren

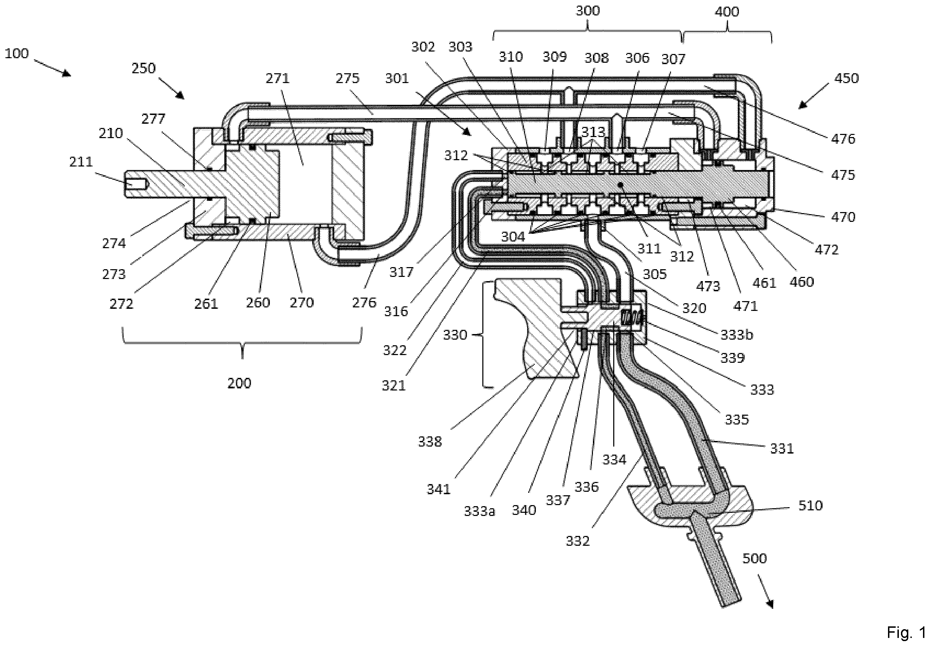

**Figur 1** zeigt einen Querschnitt einer Kraftmaschine 100. Die Kraftmaschine 100 weist ein Arbeitssystem 200, umfassend ein erstes Kolben-Zylinder-System 250, auf. Das erste Kolben-Zylinder-System 250 weist einen Arbeitskolben 260 und einen Arbeitszylinder 270 auf, wobei der Arbeitskolben 260 reversibel axial beweglich in dem Arbeitszylinder 270 angeordnet ist. Der Arbeitskolben 260 befindet sich in einer ersten Arbeitskolbenposition. Der Arbeitskolbens 260 weist einen Außenumfang auf, welcher im Wesentlichen einem Innenumfang des Arbeitszylinders 270 entspricht, wodurch der Arbeitskolben 260 den vom Arbeitszylinder 270 umgebenen Raum in einen ersten Arbeitszylinderabschnitt 271 und einen zweiten Arbeitszylinderabschnitt 272 unterteilt. Um einen Gasaustausch zwischen erstem Arbeitszylinderabschnitt 271 und zweitem Arbeitszylinderabschnitt 272 zu verhindern, weist der Arbeitskolben 260 einen Arbeitskolbendichtungsring 261 auf. Der Arbeitskolbendichtungsring 261 ist derart ausgestaltet, dass zwar ein Gasaustausch zwischen erstem Arbeitszylinderabschnitt 271 und zweitem Arbeitszylinderabschnitt 272 effektiv unterbunden wird, aber trotzdem eine Verschiebung des Arbeitskolbens 260 innerhalb des Arbeitszylinders 270 nicht beeinträchtigt wird. Dazu kann der Arbeitskolbendichtungsring 261 aus einem weichen Kunststoff, wie beispielsweise Polytetrafluorethylen, bestehen.

Der Arbeitszylinder 270 kann einteilig oder aus mehreren Bauteilen bestehend aufgebaut sein. In der gezeigten Ausgestaltungsform ist der Arbeitszylinder 270 einstückig, aber aus mehreren Bauteilen bestehend, aufgebaut. Eine Arbeitszylindervorderseite 273 des Arbeitszylinders 270 weist eine Arbeitszylinderdurchführung 274 auf, in der reversibel axial beweglich ein Arbeitselement 210 angeordnet ist. Das Arbeitselement 210 weist einen Außenumfang auf, welcher im Wesentlichen einem Innenumfang der Arbeitszylinderdurchführung 274 entspricht. Um einen Gasaustausch zwischen erstem Arbeitszylinderabschnitt 271 und einer Umgebungsatmosphäre der Kraftmaschine 100 zu verhindern, weist die Arbeitszylinderdurchführung 274 einen Arbeitszylinderdichtungsring 277 angrenzend an das Arbeitselement 210 auf.

Das Arbeitselement 210 dient dazu, die Bewegung des Arbeitskolbens 260 außerhalb der Kraftmaschine 100 mechanisch nutzbar zu machen. Dazu ist des Arbeitselement 210 mit dem Arbeitskolben 260 verbunden und erstreckt sich axial durch die Arbeitszylinderdurchführung 274 außerhalb der Kraftmaschine 100. Die Verbindung zwischen Arbeitselement 210 und Arbeitskolben 260 kann unterschiedlich ausgeformt sein. Beispielsweise können Arbeitselement 210 und Arbeitskolben 260 über eine Schraubverbindung oder Steckverbindung miteinander verbunden sein. In der gezeigten Ausgestaltungsform sind Arbeitselement 210 und Arbeitskolben 260 einteilig ausgeformt. Das Arbeitselement 210, und damit auch die Arbeitszylinderdurchführung 274, können unterschiedliche Querschnittsgeometrien, wie beispielsweise rechteckig oder dreieckig, aufweisen. Aufgrund der leichten Fertigungsweise und der geringen Gefahr für Verkantungen, ist die Querschnittsgeometrie des Arbeitselements 210 bevorzugt rund. In der gezeigten Ausgestaltungsform ist das Arbeitselement 210 als runde Stange ausgeformt.

Um die Bewegung des Arbeitskolbens 260 außerhalb der Kraftmaschine 100 mechanisch nutzbar zu machen, weist das Arbeitselement 210 eine Befestigungsvorrichtung 211 auf, um mit einem Bearbeitungsmittel (nicht gezeigt) reversibel verbunden zu werden. Die Befestigungsvorrichtung 211 kann unterschiedlich ausgeformt sein. Beispiele für Befestigungsvorrichtungen 211 sind Außengewinde, Innengewinde oder Bajonettanschlüsse. In der gezeigten Ausgestaltungsform ist die Befestigungsvorrichtung 211 als Schraubvorrichtung mit einem Innengewinde ausgeformt. Beispiele für Bearbeitungsmittel sind Sägeblätter, Bohrer, Bürsten, Fräsen und Sprühvorrichtungen, welche ein zur Befestigungsvorrichtung 211 passendes Gegenstück aufweisen, um mit dem Arbeitselement 210 verbunden werden zu können.

Die Kraftmaschine 100 weist weiterhin ein Ventilsystem 300 auf, welches dazu dient, den ersten Arbeitszylinderabschnitt 271 und den zweiten Arbeitszylinderabschnitt 272 abwechselnd mit Unterdruck zu beaufschlagen, wobei der Unterdruck durch eine Unterdruckquelle 500 zur Verfügung gestellt wird. Das Ventilsystem 300 ist als 5/2-Wegeventil ausgestaltet. Das Ventilsystem 300 weist ein rohrartiges Ventilgehäuse 301 mit einem ersten Ventilanschluss 305 auf, welcher mit der Unterdruckquelle 500 gasleitend verbindbar ist. Das Ventilgehäuse 301 weist zudem einen zweiten Ventilanschluss 306 auf, über den das Ventilgehäuse 301 mittels einer ersten Arbeitssystemzuleitung 275 mit dem zweiten Arbeitszylinderabschnitt 272 gasleitend verbunden ist. Des Weiteren weist das Ventilgehäuse 301 einen vierten Ventilanschluss 308 auf, über den das Ventilgehäuse 301 mittels einer zweiten Arbeitssystemzuleitung 276 mit dem ersten Arbeitszylinderabschnitt 271 gasleitend verbunden ist. Das Ventilgehäuse 301 ist über einen dritten Ventilanschluss 307 und über einen fünften Ventilanschluss 309 gasleitend mit der Umgebungsatmosphäre der Kraftmaschine 100 verbunden. Das Ventilgehäuse 301 kann einteilig oder aus mehreren Bauteilen zusammengesetzt aufgebaut sein. In der gezeigten Ausgestaltungsform ist das Ventilgehäuse 301 aus mehreren Bauteilen zusammengesetzt ausgebildet, wobei ein Ventilgehäuseinnenelement 303 angrenzend von einer Ventilgehäuseaußenwand 302 umgeben ist. Das Ventilgehäuseinnenelement 303 und die Ventilgehäuseaußenwand 302 bilden gemeinsam die Ventilanschlüsse 305, 306, 307, 308 und 309 aus. Um einen Gasaustausch zwischen den Ventilanschlüssen 305, 306, 307, 308 und 309 an der Grenzfläche zwischen Ventilgehäuseinnenelement 302 und Ventilgehäuseaußenwand 303 zu unterbinden, weist das Ventilgehäuseinnenelement 302 Ventilgehäusedichtungsringe 304 auf.

Das Ventilelement 310 dient dazu, den ersten Ventilanschluss 305 abwechselnd entweder mit dem zweiten Ventilanschluss 306 oder dem vierten Ventilanschluss 308 gasleitend zu verbinden. Gleichzeitig dient das Ventilelement 310 dazu, entweder den nicht mit dem ersten Ventilanschluss 305 gasleitend verbundenen zweiten Ventilanschluss 306 mit dem dritten Ventilanschluss 307 oder den nicht mit dem ersten Ventilanschluss 305 verbundenen vierten Ventilanschluss 308 mit dem fünften Ventilanschluss 309 gasleitend zu verbinden. Dies führt zu einer abwechselnden Evakuierung des Gases innerhalb des ersten Arbeitszylinderabschnitts 271 und des zweiten Arbeitszylinderabschnitts 272 und damit zu einer reversiblen axialen Bewegung des Arbeitskolbens 260 samt Arbeitselement 210 innerhalb des Arbeitszylinders 270. Das Ventilelement 310 weist hierzu einen zylinderartigen Ventilgrundkörper 311 mit vier radial um den Ventilgrundkörper 311 umlaufenden Ventilstegen 312 auf. Die Ventilstege 312 besitzen eine größere radiale Ausdehnung als der Ventilgrundkörper 311 und sind derart mit dem Ventilgrundkörper 311 verbunden, dass eine Bewegung des Ventilelements 310 nicht zu einer räumlichen Verschiebung der Ventilstege 312 auf einer Außenfläche des Ventilgrundkörpers 311 führt. Der Ventilgrundkörper 311 und die Ventilstege 312 können auf unterschiedliche Weise miteinander verbunden sein, beispielsweise form- und/oder kraftschlüssig. In der gezeigten Ausgestaltungsform sind der Ventilgrundkörper 311 und die Ventilstege 312 einteilig ausgeformt. Ein Außenumfang der Ventilstege 312 entspricht im Wesentlichen einem Innenumfang des Ventilgehäuseinnenelement 303. Jeweils räumlich zwischen zwei Ventilstegen 312 sind am Ventilelement 310 drei axial umlaufende Ventilnuten 313 ausgebildet. Die Ventilnuten 313 dienen dazu, maximal zwei benachbarte Ventilanschlüsse 305, 306, 307, 308 und 309 gasleitend miteinander zu verbinden. Jede der Ventilnuten 313 erstreckt sich in axialer Richtung des Ventilelements 310 derart, dass maximal zwei benachbarte Ventilanschlüsse 305, 306, 307, 308 und 309 von einer Ventilnut 312 gasleitend miteinander verbunden sind. Die Ventilstege 312 dienen dazu, benachbarte Ventilanschlüsse 305, 306, 307, 308 und 309 gasleitend voneinander zu trennen. Die räumliche Position des Ventilelements 310 innerhalb des Ventilgehäuses 301 bestimmt, welche benachbarten Ventilanschlüsse 305, 306, 307, 308 und 309 gasleitend miteinander verbunden oder gasleitend voneinander getrennt sind. In einer gezeigten ersten Ventilposition sind der zweite Ventilanschluss 306 und der dritte Ventilanschluss 307 sowie der erste Ventilanschluss 305 und der vierte Ventilanschluss 308 über jeweils eine Ventilnut 313 gasleitend miteinander verbunden. In einer, nicht gezeigten, zweiten Ventilposition sind der erste Ventilanschluss 305 und der zweite Ventilanschluss 306 sowie der vierte Ventilanschluss 308 und der fünfte Ventilanschluss 309 über jeweils eine Ventilnut 313 gasleitend miteinander verbunden.

Die Kraftmaschine 100 weist weiterhin ein Steuersystem 400 auf. Das Steuersystem 400 dient dazu, das Ventilelement 310 innerhalb des Ventilgehäuses 301 reversibel in die erste Ventilposition und die zweite Ventilposition zu verbringen. Das Steuersystem 400 umfasst ein zweites Kolben-Zylinder-System 450, aufweisend einen Steuerkolben 460 und einen Steuerzylinder 470, wobei der Steuerkolben 460 reversibel axial beweglich in dem Steuerzylinder 470 angeordnet ist. Der Steuerkolben 460 befindet sich in einer ersten Steuerkolbenposition. Der Steuerkolben 460 weist einen Außenumfang auf, welcher im Wesentlichen einem Innenumfang des Steuerzylinders 470 entspricht, wodurch der Steuerkolben 460 den vom Steuerzylinder 470 umgebenen Raum in einen ersten Steuerzylinderabschnitt 471 und einen zweiten Steuerzylinderabschnitt 472 unterteilt. Um einen Gasaustausch zwischen erstem Steuerzylinderabschnitt 471 und zweitem Steuerzylinderabschnitt 472 zu verhindern, weist der Steuerkolben 460 einen Steuerkolbendichtungsring 461 auf. Der Steuerkolbendichtungsring 461 ist derart ausgestaltet, dass zwar ein Gasaustausch zwischen erstem Steuerzylinderabschnitt 471 und zweitem Steuerzylinderabschnitt 472 effektiv unterbunden wird, aber trotzdem eine reversible axiale Verschiebung des Steuerkolbens 460 innerhalb des Steuerzylinders 470 nicht beeinträchtigt wird. Dazu kann der Steuerkolbendichtungsring 461 aus einem weichen Kunststoff, wie beispielsweise Polytetrafluorethylen, bestehen.

Der Steuerzylinder 470 kann einteilig oder aus mehreren Bauteilen bestehend aufgebaut sein. In der gezeigten Ausgestaltungsform ist der Steuerzylinder 470 einstückig, aber aus mehreren Bauteilen bestehend, aufgebaut. Das Steuersystem 400 und das Ventilsystem 300 können einstückig aufgebaut sein oder aus zwei getrennten Systemen bestehen. In der gezeigten Ausgestaltungsform ist das Steuersystem 400 einstückig mit dem Ventilsystem 300 ausgestaltet. Der Steuerzylinder 470 weist an der dem Ventilsystem 300 zugewandten Seite eine Steuerzylinderdurchführung 473 auf, durch welche das Ventilelement 310 mit dem Steuerkolben 460 verbunden ist. Das Ventilelement 310 und der Steuerkolben 460 können auf unterschiedliche Weisen, beispielsweise form- und/oder kraftschlüssig, miteinander verbunden sein. In der gezeigten Ausgestaltungsform sind Ventilelement 310 und Steuerkolben 460 einteilig ausgestaltet. Eine Bewegung des Steuerkolbens 460 führt unmittelbar zu einer gleichartigen Bewegung des Ventilelements 310. Um einen Gasaustausch zwischen Ventilsystem 300 und Steuersystem 400 zu unterbinden, weist die Steuerzylinderdurchführung 473 einen Innenumfang auf, welcher im Wesentlichen dem Außenumfang des Ventilelements 310 entspricht.

Das Steuersystem 400 ist gasleitend mit dem Ventilsystem 300 verbunden, wobei der erste Steuerzylinderabschnitt 471 gasleitend über eine erste Steuersystemzuleitung 475 mit dem zweiten Ventilanschluss 306 und der zweite Steuerzylinderabschnitt 472 gasleitend über eine zweite Steuersystemzuleitung 476 mit dem vierten Ventilanschluss 308 verbunden ist. Die erste Arbeitssystemzuleitung 275 und die erste Steuersystemzuleitung 475 sowie die zweite Arbeitssystemzuleitung 276 und die zweite Steuersystemzuleitung 476 sind jeweils ein über einen gemeinsamen Zuleitungsabschnitt mit dem entsprechenden Ventilanschluss verbunden. Dies erleichtert die Verschaltung des Arbeitssystems 200 und des Steuersystems 400 mit dem Ventilsystem 300.

Der erste Ventilanschluss 305 ist gasleitend über eine erste Ventilsystemzuleitung 320 mit einem Ventilschalter 330 verbunden. Der Ventilschalter 330 ist über eine erste Ventilschalterzuleitung 331 gasleitend mit der Unterdruckquelle 500 verbunden. Der Ventilschalter 330 weist ein Ventilschaltergehäuse 333 und ein axial im Ventilschaltergehäuse 333 bewegliches Ventilschalterelement 334 auf. Das Ventilschaltergehäuse 333 weist in axialer Richtung ein offenes Ende 333a und ein geschlossenes Ende 333b auf. Der Ventilschalter 330 verhindert in einer gezeigten ersten Ventilschalterposition einen Gasaustausch zwischen Ventilsystem 300 und Unterdruckquelle 500. Dazu weist das Ventilschalterelement 334 einen ersten Ventilschaltersteg 335 auf, welche einen Außenumfang aufweist, welcher im Wesentlichen einem Innenumfang des Ventilschaltergehäuses 333 entspricht. Durch den ersten Ventilschaltersteg 335 wird eine Verbindung zwischen der ersten Ventilsystemzuleitung 320 und der ersten Ventilschalterzuleitung 331 in der gezeigten ersten Ventilschalterposition gasleitend verschlossen.

Der Ventilschalter 330, und insbesondere das Ventilschalterelement 334, kann durch einen externen Krafteintrag durch einen Anwender der Kraftmaschine 100 aus der gezeigten ersten Ventilschalterposition in eine, nicht gezeigte, zweite Ventilschalterposition verschoben werden. Um den externen Krafteintrag auf das Ventilschalterelement 334 zu übertragen, weist der Ventilschalter 330 einen Ventilschalterabzug 338 auf, welcher mit dem Ventilschalterelement 334 durch das offene Ende 333a des Ventilschaltergehäuses 333 verbunden ist. Die Verbindung zwischen Ventilschalterabzug 338 und Ventilschalterelement 334 kann unterschiedlich ausgestaltet sein. Beispielsweise können Ventilschalterabzug 338 und Ventilschalterelement 334 einteilig ausgestaltet sein. In der gezeigten Ausgestaltungsform sind Ventilschalterabzug 338 und Ventilschalterelement 334 einstückig, aber aus zwei formschlüssig verbundenen Bauteilen bestehend, aufgebaut.

Das Ventilschaltergehäuse 333 weist an dem geschlossenen Ende 333b eine Feder 339 auf, welche das Ventilschalterelement 334 in Richtung des offenen Endes 333a des Ventilgehäuses 333 verschiebt. Die Feder 339 dient dazu, den Ventilschalter 330, bei nicht vorhandenem externen Krafteintrag durch den Anwender der Kraftmaschine 100, in der ersten Ventilschalterposition zu halten. Um ein vollständiges Hinausschieben des Ventilschalterelements 334 aus dem offenen Ende 333a des Ventilschaltergehäuse 333 zu unterbinden, weist das Ventilschaltergehäuse 333 an dem offenen Ende 333a einen Stift 340 auf. Der Stift 340 erstreckt sich in den Innenraum des Ventilschaltergehäuses 333 und wirkt derart mit einer radial umlaufenden Ventilschalterelementvertiefung 341 zusammen, dass das Ventilschalterelement 334 nicht durch die Feder 339 aus dem Ventilschaltergehäuse 333 geschoben wird.

Der Ventilschalter 340 ist neben der ersten Ventilsystemzuleitung 320 auch über eine zweite Ventilsystemzuleitung 321 und eine dritte Ventilsystemzuleitung 322 gasleitend mit dem Ventilsystem 300 verbunden. Die zweite Ventilsystemzuleitung 321 dient dazu, das Ventilelement 310, bei nicht betätigtem Ventilschalter 330, durch Beaufschlagung mit von der Unterdruckquelle 500 bereitgestelltem Unterdruck in der ersten Ventilposition zu halten. Dadurch wird die gesamte Kraftmaschine 100 in einem definierten Ausgangzustand gehalten, was beispielsweise die Befestigung eines Bearbeitungsmittels an dem Arbeitselement 210 vereinfacht. Die dritte Ventilsystemzuleitung 322 dient dazu, das Ventilsystem 300 mit der Umgebungsatmosphäre der Kraftmaschine 100 gasleitend zu verbinden. Ohne eine gasleitende Verbindung mit der Umgebungsatmosphäre der Kraftmaschine 100 ist eine reversible axiale Verschiebung des Ventilelements 210 innerhalb des Ventilgehäuses 301 nicht möglich.

Um den Ventilschalter 330 über die zweite Ventilsystemzuleitung 321 und die dritte Ventilsystemzuleitung 322 gasleitend mit dem Ventilsystem zu verbinden, weist das Ventilgehäuse 301 an einer Stirnseite eine erste Ventilabschnittdurchführung 316 und eine zweite Ventilabschnittdurchführung 317 auf. Die erste Ventilabschnittdurchführung 316 ist über die zweite Ventilsystemzuleitung 321 und die zweite Ventilabschnittdurchführung 317 ist über die zweite Ventilsystemzuleitung 320 mit dem Ventilschaltergehäuse 333 gasleitend verbunden. Das Ventilschaltergehäuse 333 ist weiterhin über eine zweite Ventilschalterzuleitung 332 mit der Unterdruckquelle 500 gasleitend verbunden. Die erste Ventilschalterzuleitung 331 und die zweite Ventilschalterzuleitung 332 sind über ein Dreiwegeventil 510 mit der Unterdruckquelle verbunden. Ein Vorteil des Dreiwegeventils 510 ist die vereinfachte gasleitende Verbindung der Kraftmaschine 100 mit der Unterdruckquelle 500, da auf diese Weise die Kraftmaschine 100 mit nur einer einzigen gasleitende Verbindung mit der Unterdruckquelle 500 betreibbar ist.

In der gezeigten ersten Ventilschalterposition grenzt die zweite Ventilschalterzuleitung 322 an eine radial umlaufende Ventilschalternut 336 des Ventilschalterelements 334. Die Ventilschalternut 336 stellt eine gasleitende Verbindung zwischen zweiter Ventilsystemzuleitung 321 und zweiter Ventilschalterzuleitung 332 her, so dass in der ersten Ventilschalterstellung das Ventilelement 310 durch die Einwirkung von Unterdruck formschlüssig an die erste Ventilabschnittdurchführung anliegt. In der ersten Ventilschalterposition verbleibt das Ventilelement 310 in der ersten Ventilposition.

In der gezeigten ersten Ventilschalterposition grenzt die dritte Ventilsystemzuleitung 322 am Ventilschalter 330 an einen zweiten Ventilschaltersteg 337 des Ventilschalterelements 334, so dass eine gasleitende Verbindung zu der Umgebungsatmosphäre der Kraftmaschine 100 verschlossen ist.

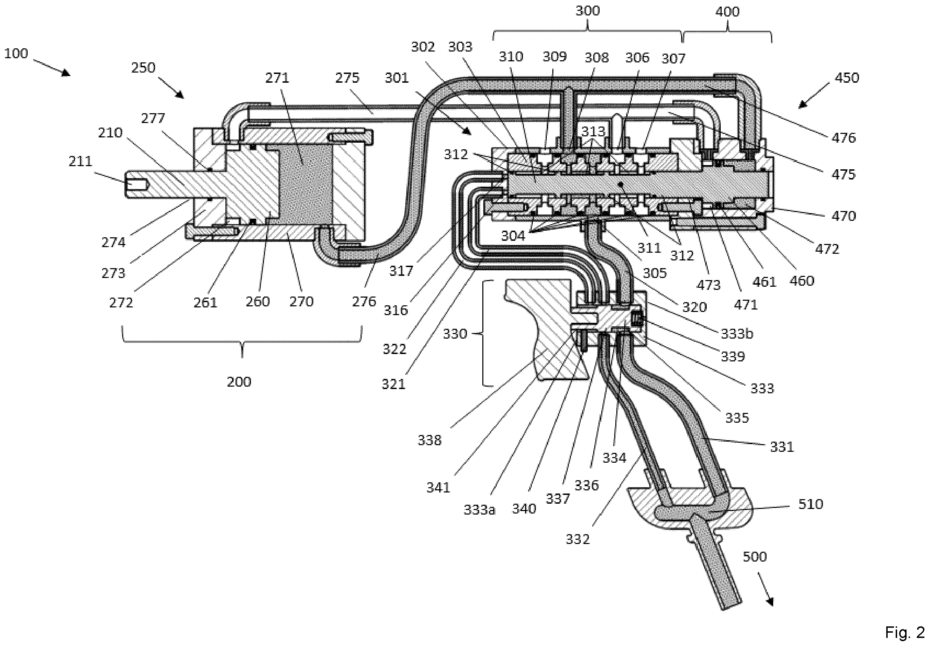

**Figur 2** zeigt die Kraftmaschine 100 aus Figur 1 in einer ersten Arbeitsposition. Der Ventilschalterabzug 338, und damit das Ventilschalterelement 334 ist in Richtung des geschlossenen Endes 333b des Ventilschaltergehäuses 333 reversibel axial verschoben und befindet sich somit in der zweiten Ventilschalterposition. Der zweite Ventilschaltersteg 337 des Ventilschalterelements 334 ist in der zweiten Ventilschalterposition angrenzend an die zweite Ventilsystemzuleitung 321 positioniert und unterbricht somit die gasleitende Verbindung zwischen Unterdruckquelle 500 und zweiter Ventilsystemzuleitung 321. Zudem ist die dritte Ventilsystemzuleitung 322 in der zweiten Ventilschalterposition am Ventilschalter 330 angrenzend an die Ventilschalterelementvertiefung 341 positioniert, wodurch die zweite Ventilabschnittsdurchführung 317 gasleitend mit der Umgebungsatmosphäre der Kraftmaschine 100 verbunden ist.

Durch das Verbringen des Ventilschalters 330 in die zweite Ventilschalterposition ist das Ventilelement 210 nicht mehr in der ersten Ventilposition fixiert und kann innerhalb des Ventilgehäuses 301 axial verschoben werden. Weiterhin ist in der zweiten Ventilschalterposition der erste Ventilanschluss 305 über die erste Ventilsystemzuleitung 320, die axial verschobene Ventilschalternut 336 und die erste Ventilschalterzuleitung 331 gasleitend mit der Unterdruckquelle 500 verbunden. Der erste Ventilschalteranschluss 305 ist in der vorliegenden ersten Ventilposition gasleitend über eine Ventilnut 313 mit dem vierten Ventilanschluss 308 verbunden, so dass sowohl der erste Arbeitszylinderabschnitt 271 über die zweite Arbeitssystemzuleitung 276, als auch der zweite Steuerzylinderabschnitt 472 über die zweite Steuersystemzuleitung 476 gasleitend mit der Unterdruckquelle 500 verbunden ist.

Zugleich ist in der ersten Ventilposition der dritte Ventilanschluss 307 über eine Ventilnut 313 gasleitend mit dem zweiten Ventilanschluss 306 verbunden, so dass sowohl der zweite Arbeitszylinderabschnitt 272 über die erste Arbeitssystemzuleitung 275, als auch der erste Steuerzylinderabschnitt 471 über die erste Steuersystemzuleitung 475 gasleitend mit der Umgebungsatmosphäre der Kraftmaschine 100 verbunden sind.

Dies hat insgesamt sowohl eine Verschiebung des Arbeitskolbens 260 aus der ersten Arbeitskolbenposition in eine zweite Arbeitskolbenposition, als auch eine Verschiebung des Steuerkolbens 460 aus der ersten Steuerkolbenposition in eine zweite Steuerkolbenposition zur Folge. Das Verbringen des Steuerkolbens 460 aus der ersten Steuerkolbenposition in die zweite Steuerkolbenposition führt unmittelbar zu einer Verschiebung des Ventilelements 310 aus der ersten Ventilposition in die zweite Ventilposition.

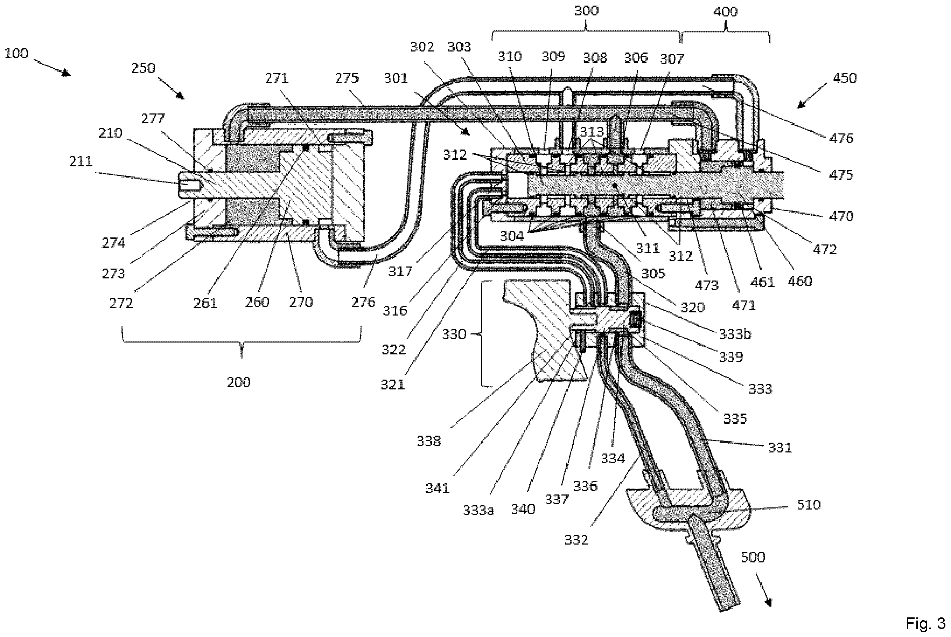

**Figur 3** zeigt die Kraftmaschine 100 aus Figur 1 und Figur 2 in einer zweiten Arbeitsposition. Der Ventilschalter 330 befindet sich wie in Figur 2 in der zweiten Ventilschalterposition, so dass die Unterdruckquelle 500 gasleitend mit dem ersten Ventilanschluss 305 in Verbindung steht. Der Steuerkolben 460 befindet sich in der zweiten Steuerkolbenposition und damit auch das Ventilelement 210 in der zweiten Ventilposition.

Das Ventilelement 310 in der zweiten Ventilposition verbindet den ersten Ventilanschluss 305 gasleitend über eine Ventilnut 313 mit dem zweiten Ventilanschluss 306. Somit ist sowohl der zweite Arbeitszylinderabschnitt 272 über die erste Arbeitssystemzuleitung 275, als auch der erste Steuerzylinderabschnitt 471 über die erste Steuersystemzuleitung 475 gasleitend mit der Unterdruckquelle 500 verbunden. Zudem ist der fünfte Ventilanschluss 309 über eine Ventilnut 313 gasleitend mit dem vierten Ventilanschluss 308 verbunden, wodurch sowohl der erste Arbeitszylinderabschnitt 271 über die zweite Arbeitssystemzuleitung 276, als auch der zweite Steuerzylinderabschnitt 472 über die zweite Steuersystemzuleitung 476 gasleitend mit der Umgebungsatmosphäre der Kraftmaschine 100 verbunden sind.

Dies hat insgesamt sowohl eine Verschiebung des Arbeitskolbens 260 aus der zweiten Arbeitskolbenposition in die erste Arbeitskolbenposition, als auch eine Verschiebung des Steuerkolbens 460 aus der zweiten Steuerkolbenposition in die erste Steuerkolbenposition zur Folge. Das Verbringen des Steuerkolbens 460 aus der zweiten Steuerkolbenposition in die erste Steuerkolbenposition führt unmittelbar zu einer Verschiebung des Ventilelements 310 aus der ersten Ventilposition in die zweite Ventilposition, womit die Kraftmaschine 100 wieder in den die erste Arbeitsposition aus Figur 2 verschoben wird.

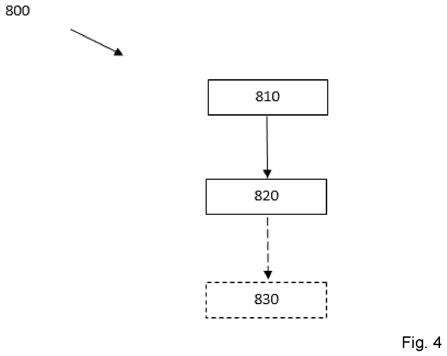

**Figur 4** zeigt ein Flussdiagramm eines Verfahrens 800 zur Behandlung eines Säugetiers, insbesondere eines Menschen, umfassend die Schritte 810 und 820, sowie den optionalen Schritt 830. In Schritt 810 wird eine medizinische Vorrichtung, aufweisend die Kraftmaschine 100, bereitgestellt. In Schritt 820 wird die medizinische Vorrichtung zum Bearbeiten, insbesondere zum Sägen, Fräsen, Bürsten, Bohren und/oder Besprühen, von Knochenmaterial verwendet. In einem optionalen Schritt 830 wird die medizinische Vorrichtung nach dem Bearbeiten von Knochenmaterial, vorzugsweise ohne vorherige Reinigungs- und/oder Desinfektionsschritte, entsorgt.

Die in den Ansprüchen, der Beschreibung und in den Figuren offenbarten Merkmale können für verschiedene Ausgestaltungsformen der beanspruchten Erfindung sowohl getrennt als auch in beliebiger Kombination miteinander wesentlich sein. Die für die Kraftmaschine offenbarten Merkmale sind auch für die medizinische Vorrichtung offenbart und umgekehrt.

### Bezugszeichen

- 100: Kraftmaschine
- 200: Arbeitssystem
- 210: Arbeitselement
- 211: Befestigungsvorrichtung
- 250: erstes Kolben-Zylinder-System
- 260: Arbeitskolben
- 261: Arbeitskolbendichtungsring
- 270: Arbeitszylinder
- 271: erstes Arbeitszylinderabschnitt
- 272: zweiter Arbeitszylinderabschnitt
- 273: Arbeitszylindervorderseite
- 274: Arbeitszylinderdurchführung
- 275: erste Arbeitssystemzuleitung
- 276: zweite Arbeitssystemzuleitung
- 277: Arbeitszylinderdichtungsring
- 300: Ventilsystem
- 301: Ventilgehäuse
- 302: Ventilgehäuseaußenwand
- 303: Ventilgehäuseinnenelement
- 304: Ventilgehäusedichtungsringe
- 305: erster Ventilanschluss
- 306: zweiter Ventilanschluss
- 307: dritter Ventilanschluss
- 308: vierter Ventilanschluss
- 309: fünfter Ventilanschluss
- 310: Ventilelement
- 311: Ventilgrundkörper
- 312: Ventilsteg
- 313: Ventilnut
- 316: erste Ventilabschnittdurchführung
- 317: zweite Ventilabschnittdurchführung
- 320: erste Ventilsystemzuleitung
- 321: zweite Ventilsystemzuleitung
- 322: dritte Ventilsystemzuleitung
- 330: Ventilschalter
- 331: erste Ventilschalterzuleitung
- 332: zweite Ventilschalterzuleitung
- 333: Ventilschaltergehäuse
- 333a: offenes Ende des Ventilschaltergehäuses
- 333b: geschlossenes Ende des Ventilschaltergehäuses
- 334: Ventilschalterelement
- 335: erster Ventilschaltersteg
- 336: Ventilschalternut
- 337: zweiter Ventilschaltersteg
- 338: Ventilschalterabzug
- 339: Feder
- 340: Stift
- 341: Ventilschalterelementvertiefung
- 400: Steuersystem
- 450: zweites Kolben-Zylinder-System
- 460: Steuerkolben
- 461: Steuerkolbendichtungsring
- 470: Steuerzylinder
- 471: erster Steuerzylinderabschnitt
- 472: zweiter Steuerzylinderabschnitt
- 473: Steuerzylinderdurchführung
- 475: erste Steuersystemzuleitung
- 476: zweite Steuersystemzuleitung
- 500: Unterdruckquelle
- 510: Dreiwegeventil
- 800: Verfahren zur Behandlung eines Säugetiers
- 810: Bereitstellen einer medizinischen Vorrichtung
- 820: Bearbeiten von Knochenmaterial mittels der medizinischen Vorrichtung
- 830: Entsorgen der medizinischen Vorrichtung

## Patentansprüche

1. Kraftmaschine (100) umfassend
ein Arbeitssystem (200), aufweisend ein erstes Kolben-Zylinder-System (250), umfassend einen Arbeitskolben (260) und einen Arbeitszylinder (270), wobei der Arbeitskolben (260) den Arbeitszylinder (270) in einen ersten Arbeitszylinderabschnitt (271) und einen zweiten Arbeitszylinderabschnitt (272) unterteilt,
ein Ventilsystem (300), aufweisend einen ersten Ventilanschluss (305) und ein Ventilelement (310), wobei das Ventilsystem (300) und das Arbeitssystem (200) gasleitend verbunden sind,
der erste Ventilanschluss (305) mit einer Unterdruckquelle (500) verbindbar ist und
das Ventilelement (310) derart beweglich in dem Ventilsystem (300) angeordnet ist, dass das Ventilelement (310) in einer ersten Ventilposition den ersten Ventilanschluss (305) mit dem ersten Arbeitszylinderabschnitt (271) und in einer zweiten Ventilposition den ersten Ventilanschluss (305) mit dem zweiten Arbeitszylinderabschnitt (272) gasleitend verbindet,
ein Steuersystem (400), wobei das Steuersystem (400) ein zweites Kolben-Zylinder-System (450), aufweisend einen Steuerkolben (460) und einen Steuerzylinder (470), umfasst,
wobei der Steuerkolben (460) derart mit dem Ventilelement (310) verbunden ist, dass eine zyklische Bewegung des Steuerkolbens (460) im Steuerzylinder (470) das Ventilelement (310) zwischen der ersten Ventilposition und der zweiten Ventilposition bewegt,
wobei der erste Ventilanschluss (305) über eine erste Ventilsystemzuleitung (320) mit einem Ventilschalter (330) verbunden ist, wobei der Ventilschalter (330) die erste Ventilsystemzuleitung (320) in einer ersten Ventilschalterposition (331) reversibel gasleitend verschließt und in einer zweiten Ventilschalterposition (332) die erste Ventilsystemzuleitung (320) reversibel gasleitend öffnet,
**dadurch gekennzeichnet, dass**
zwischen Ventilsystem (300) und Ventilschalter (330) eine zweite Ventilsystemzuleitung (321) und eine dritte Ventilsystemzuleitung (322) angeordnet ist, wobei der Ventilschalter (330) in der ersten Ventilschalterposition das Ventilsystem (300) über die zweite Ventilsystemzuleitung (321) mit der Unterdruckquelle (500) gasleitend verbindet und in der zweiten Ventilschalterposition das Ventilsystem (300) über die dritte Ventilsystemzuleitung (322) mit der Umgebungsatmosphäre der Kraftmaschine (100) gasleitend verbindet.

2. Kraftmaschine (100) nach Anspruch 1, **dadurch gekennzeichnet, dass** eine Bewegung des Steuerkolbens (460) in eine erste Steuerkolbenposition das Ventilelement (310) in die erste Ventilposition und eine Bewegung des Steuerkolbens (460) in eine zweite Steuerkolbenposition das Ventilelement (310) in die zweite Ventilposition verbringt.

3. Kraftmaschine (100) nach Anspruch 1 oder 2, **dadurch gekennzeichnet, dass** der Steuerkolben (460) und das Ventilelement (310) mechanisch gekoppelt sind.

4. Kraftmaschine (100) nach einem der vorhergehenden Ansprüche, **dadurch gekennzeichnet, dass** der Steuerkolben (460) und das Ventilelement (310) einteilig ausgestaltet sind.

5. Kraftmaschine (100) nach einem der vorhergehenden Ansprüche, **dadurch gekennzeichnet, dass** das Steuersystem (400) gasleitend mit dem Ventilsystem (300) verbunden ist.

6. Kraftmaschine (100) nach Anspruch 5, **dadurch gekennzeichnet, dass** der Steuerkolben (460) den Steuerzylinder (470) in einen ersten Steuerzylinderabschnitt (471) und einen zweiten Steuerzylinderabschnitt (472) unterteilt, wobei der erste Steuerzylinderabschnitt (471) und der zweite Steuerzylinderabschnitt (472) gasleitend mit dem Ventilsystem (300) verbunden ist.

7. Kraftmaschine (100) nach Anspruch 6, **dadurch gekennzeichnet, dass** das Ventilelement (310) in der ersten Ventilposition den ersten Ventilanschluss (305) und den zweiten Steuerzylinderabschnitt (472) und in der zweiten Ventilposition den ersten Ventilanschluss (305) und den ersten Steuerzylinderabschnitt (471) gasleitend verbindet.

8. Kraftmaschine (100) nach Anspruch 6 oder 7, **dadurch gekennzeichnet, dass** das Ventilsystem (300) als 5/2-Wegeventil ausgestaltet ist.

9. Kraftmaschine (100) nach Anspruch 5, **dadurch gekennzeichnet, dass** der Steuerkolben (460) den Steuerzylinder (470) in einen ersten Steuerzylinderabschnitt (471) und einen zweiten Steuerzylinderabschnitt (472) unterteilt, wobei der erste Steuerzylinderabschnitt (471) oder der zweite Steuerzylinderabschnitt (472) gasleitend mit dem Ventilsystem (300) verbunden ist.

10. Kraftmaschine (100) nach Anspruch 9, **dadurch gekennzeichnet, dass** der mit dem Ventilsystem (300) gasleitend verbundene Steuerzylinderabschnitt (471, 472) ein Energiespeicherelement enthält.

11. Kraftmaschine (100) nach Anspruch 10, **dadurch gekennzeichnet, dass** das Energiespeicherelement eine Feder ist.

12. Kraftmaschine (100) nach den Ansprüchen 6 bis 11, **dadurch gekennzeichnet, dass** das Arbeitssystem (200) über eine erste Arbeitssystemzuleitung (275) und das Steuersystem (400) über eine erste Steuersystemzuleitung (475) gasleitend mit dem Ventilsystem (300) verbunden ist, wobei der Quotient der Querschnittsöffnung der ersten Arbeitssystemzuleitung (275) zur Querschnittsöffnung der ersten Steuersystemzuleitung (475) gleich oder größer dem Quotienten der Summe der Volumina des ersten Arbeitszylinderabschnitts (271) und des zweiten Arbeitszylinderabschnitts (272) zur Summe der Volumina des ersten Steuerzylinderabschnitts (471) und des zweiten Steuerzylinderabschnitts (472) ist.

13. Kraftmaschine (100) nach einem der vorhergehenden Ansprüche, **dadurch gekennzeichnet, dass** das Arbeitssystem (200) ein Arbeitselement (210) aufweist.

14. Kraftmaschine (100) nach Anspruch 13, **dadurch gekennzeichnet, dass** das Arbeitselement (210) kraft- und/oder formschlüssig mit dem Arbeitskolben (260) verbunden ist oder dass das Arbeitselement (210) und der Arbeitskolben (260) einteilig ausgestaltet sind.

15. Kraftmaschine (100) nach Anspruch 13 oder 14, **dadurch gekennzeichnet, dass** das Arbeitselement (210) mit einem Bearbeitungsmittel, insbesondere zum Sägen, Fräsen, Bürsten, Bohren oder Besprühen von Knochenmaterial, ausstattbar ist.

16. Kraftmaschine (100) nach einem der vorhergehenden Ansprüche, **dadurch gekennzeichnet, dass** die Kraftmaschine (100) aus Kunststoff, Metall oder aus einer Kombination von Kunststoff und Metall gefertigt ist.

17. Kraftmaschine (100) nach einem der vorhergehenden Ansprüche, **dadurch gekennzeichnet, dass** der Arbeitskolben (260) und der Steuerkolben (460) derart gekoppelt sind, dass eine Steuerfrequenz des Steuerkolbens (460) im Wesentlichen einflussfrei von einer Arbeitsfrequenz des Arbeitskolbens (260) ist.

18. Kraftmaschine (100) nach einem der vorhergehenden Ansprüche, **dadurch gekennzeichnet, dass** der Arbeitskolben (260) einen Durchmesser zwischen 10 mm und 40 mm, bevorzugt zwischen 15 mm und 35 mm, noch bevorzugter zwischen 20 mm und 30 mm, aufweist.

19. Medizinische Vorrichtung, insbesondere zum Sägen, Fräsen, Bürsten, Bohren oder Besprühen von Knochenmaterial, **dadurch gekennzeichnet, dass** die medizinische Vorrichtung eine Kraftmaschine (100) nach einem der vorhergehenden Ansprüche aufweist.

20. Medizinische Vorrichtung nach Anspruch 19, **dadurch gekennzeichnet, dass** die medizinische Vorrichtung einen Griff aufweist, wobei das Ventilsystem (300) innerhalb des Griffs angeordnet ist.

## Claims

1. A power-generating machine (100) comprising:
a working system (200) having a first piston-cylinder system (250) comprising a working piston (260) and a working cylinder (270), the working piston (260) dividing the working cylinder (270) into a first working cylinder portion (271) and a second working cylinder portion (272),
a valve system (300) having a first valve port (305) and a valve element (310), the valve system (300) and the working system (200) being connected in a gas-conducting manner, the first valve port (305) being connectable to a negative pressure source (500) and the valve element (310) being movably arranged in the valve system (300) such that the valve element (310) connects the first valve port (305) to the first working cylinder portion (271) in a first valve position and the first valve port (305) to the second working cylinder portion (272) in a second valve position in a gas-conducting manner,
a control system (400), the control system (400) comprising a second piston-cylinder system (450) having a control piston (460) and a control cylinder (470),
the control piston (460) being connected to the valve element (310) such that a cyclical movement of the control piston (460) in the control cylinder (470) moves the valve element (310) between the first valve position and the second valve position, the first valve port (305) being connected to a valve switch (330) via a first valve system supply line (320), the valve switch (330) reversibly closing the first valve system supply line (320) in a gas-conducting manner in a first valve switch position (331) and reversibly opening the first valve system supply line (320) in a gas-conducting manner in a second valve switch position (332),
**characterized in that**
a second valve system supply line (321) and a third valve system supply line (322) are arranged between the valve system (300) and the valve switch (330), the valve switch (330) connecting the valve system (300) to the negative pressure source (500) in a gas-conducting manner via the second valve system supply line (321) in the first valve switch position and connecting the valve system (300) to the ambient atmosphere of the power-generating machine (100) in a gas-conducting manner via the third valve system supply line (322) in the second valve switch position.

2. The power-generating machine (100) according to claim 1, **characterized in that** a movement of the control piston (460) into a first control piston position passes the valve element (310) into the first valve position and a movement of the control piston (460) into a second control piston position passes the valve element (310) into the second valve position.

3. The power-generating machine (100) according to either claim 1 or claim 2, **characterized in that** the control piston (460) and the valve element (310) are mechanically coupled.

4. The power-generating machine (100) according to any of the preceding claims, **characterized in that** the control piston (460) and the valve element (310) are designed in one piece.

5. The power-generating machine (100) according to any of the preceding claims, **characterized in that** the control system (400) is connected to the valve system (300) in a gas-conducting manner.

6. The power-generating machine (100) according to claim 5, **characterized in that** the control piston (460) divides the control cylinder (470) into a first control cylinder portion (471) and a second control cylinder portion (472), the first control cylinder portion (471) and the second control cylinder portion (472) being connected to the valve system (300) in a gas-conducting manner.

7. The power-generating machine (100) according to claim 6, **characterized in that** the valve element (310) connects the first valve port (305) and the second control cylinder portion (472) in the first valve position and the first valve port (305) and the first control cylinder portion (471) in the second valve position in a gas-conducting manner.

8. The power-generating machine (100) according to either claim 6 or claim 7, **characterized in that** the valve system (300) is designed as a 5/2 directional control valve.

9. The power-generating machine (100) according to claim 5, **characterized in that** the control piston (460) divides the control cylinder (470) into a first control cylinder portion (471) and a second control cylinder portion (472), the first control cylinder portion (471) or the second control cylinder portion (472) being connected to the valve system (300) in a gas-conducting manner.

10. The power-generating machine (100) according to claim 9, **characterized in that** the control cylinder portion (471, 472) which is connected to the valve system (300) in a gas-conducting manner contains an energy storage element.

11. The power-generating machine (100) according to claim 10, **characterized in that** the energy storage element is a spring.

12. The power-generating machine (100) according to claims 6 to 11, **characterized in that** the working system (200) is connected via a first working system supply line (275) and the control system (400) via a first control system supply line (475) to the valve system (300) in a gas-conducting manner, the quotient of the cross-sectional opening of the first working system supply line (275) to the cross-sectional opening of the first control system supply line (475) being greater than or equal to the quotient of the sum of the volumes of the first working cylinder portion (271) and the second working cylinder portion (272) to the sum of the volumes of the first control cylinder portion (471) and the second control cylinder portion (472).

13. The power-generating machine (100) according to any of the preceding claims, **characterized in that** the working system (200) comprises a working element (210).

14. The power-generating machine (100) according to claim 13, **characterized in that** the working element (210) is connected to the working piston (260) in a force-fitting and/or form-fitting manner, or **in that** the working element (210) and the working piston (260) are designed in one piece.

15. The power-generating machine (100) according to either claim 13 or claim 14, **characterized in that** the working element (210) can be equipped with a processing means, in particular for sawing, milling, brushing, drilling or spraying bone material.

16. The power-generating machine (100) according to any of the preceding claims, **characterized in that** the power-generating machine (100) is made of plastics material, metal or a combination of plastics material and metal.

17. The power-generating machine (100) according to any of the preceding claims, **characterized in that** the working piston (260) and the control piston (460) are coupled such that a control frequency of the control piston (460) is substantially free of influence from a working frequency of the working piston (260).

18. The power-generating machine (100) according to any of the preceding claims, **characterized in that** the working piston (260) has a diameter between 10 mm and 40 mm, preferably between 15 mm and 35 mm, more preferably between 20 mm and 30 mm.

19. A medical device, in particular for sawing, milling, brushing, drilling or spraying bone material, **characterized in that** the medical device comprises a power-generating machine (100) according to any of the preceding claims.

20. The medical device according to claim 19, **characterized in that** the medical device has a handle, the valve system (300) being arranged within the handle.

## Revendications

1. Moteur (100), comprenant
un système de travail (200) présentant un premier système à piston et cylindre (250) qui comprend un piston de travail (260) et un cylindre de travail (270), le piston de travail (260) divisant le cylindre de travail (270) en une première section de cylindre de travail (271) et une seconde section de cylindre de travail (272),
un système de soupape (300) présentant un premier raccord de soupape (305) et un élément de soupape (310), le système de soupape (300) et le système de travail (200) étant reliés selon une liaison de transmission de gaz,
le premier raccord de soupape (305) pouvant être relié à une source de dépression (500) et l'élément de soupape (310) étant disposé de façon mobile dans le système de soupape (300) de sorte que, dans une première position de soupape, l'élément de soupape (310) relie, selon une liaison de transmission de gaz, le premier raccord de soupape (305) à la première section de cylindre de travail (271) et, dans une seconde position de soupape, relie, selon une liaison de transmission de gaz, le premier raccord de soupape (305) à la seconde section de cylindre de travail (272),
un système de commande (400), le système de commande (400) comprenant un second système à piston et cylindre (450) présentant un piston de commande (460) et un cylindre de commande (470),
le piston de commande (460) étant relié à l'élément de soupape (310) de sorte qu'un mouvement cyclique du piston de commande (460) dans le cylindre de commande (470) déplace l'élément de soupape (310) entre la première position de soupape et la seconde position de soupape, le premier raccord de soupape (305) étant relié à un commutateur de soupape (330) par l'intermédiaire d'une première conduite de système de soupape (320), le commutateur de soupape (330) fermant la première conduite de système de soupape (320) dans une première position de commutateur de soupape (331), de manière réversible et selon une liaison de transmission de gaz, et ouvrant la première conduite de système de soupape (320) dans une seconde position de commutateur de soupape (332), de manière réversible et selon une liaison de transmission de gaz,
**caractérisé en ce que,**
entre le système de soupape (300) et le commutateur de soupape (330), sont disposées une deuxième conduite de système de soupape (321) et une troisième conduite de système de soupape (322), le commutateur de soupape (330) reliant, dans une première position de commutateur de soupape et selon une liaison de transmission de gaz, le système de soupape (300) à la source de dépression (500) par l'intermédiaire de la deuxième conduite de système de soupape (321) et mettant en communication, dans la seconde position de commutateur de soupape et selon une liaison de transmission de gaz, le système de soupape (300) avec l'atmosphère ambiante du moteur (100) par l'intermédiaire de la troisième conduite de système de soupape (322).

2. Moteur (100) selon la revendication 1, **caractérisé en ce qu'**un mouvement du piston de commande (460) dans une première position de piston de commande déplace l'élément de soupape (310) dans la première position de soupape, et un mouvement du piston de commande (460) dans une seconde position de piston de commande déplace l'élément de soupape (310) dans la seconde position de soupape.

3. Moteur (100) selon la revendication 1 ou 2, **caractérisé en ce que** le piston de commande (460) et l'élément de soupape (310) sont accouplés mécaniquement.

4. Moteur (100) selon l'une quelconque des revendications précédentes, **caractérisé en ce que** le piston de commande (460) et l'élément de soupape (310) sont réalisés d'un seul tenant.

5. Moteur (100) selon l'une quelconque des revendications précédentes, **caractérisé en ce que** le système de commande (400) est relié au système de soupape (300) selon une liaison de transmission de gaz.

6. Moteur (100) selon la revendication 5, **caractérisé en ce que** le piston de commande (460) divise le cylindre de commande (470) en une première section de cylindre de commande (471) et une seconde section de cylindre de commande (472), la première section de cylindre de commande (471) et la seconde section de cylindre de commande (472) étant reliées au système de soupape (300) selon une liaison de transmission de gaz.

7. Moteur (100) selon la revendication 6, **caractérisé en ce que,** dans la première position de soupape, l'élément de soupape (310) relie, selon une liaison de transmission de gaz, le premier raccord de soupape (305) et la seconde section de cylindre de commande (472) et relie, selon une liaison de transmission de gaz, le premier raccord de soupape (305) et la première section de cylindre de commande (471), dans la seconde position de soupape.

8. Moteur (100) selon la revendication 6 ou 7, **caractérisé en ce que** le système de soupape (300) est réalisé sous forme de soupape de distribution à 5 voies/2 positions.

9. Moteur (100) selon la revendication 5, **caractérisé en ce que** le piston de commande (460) divise le cylindre de commande (470) en une première section de cylindre de commande (471) et une seconde section de cylindre de commande (472), la première section de cylindre de commande (471) ou la seconde section de cylindre de commande (472) étant reliée au système de soupape (300) selon une liaison de transmission de gaz.

10. Moteur (100) selon la revendication 9, **caractérisé en ce que** la section de cylindre de commande (471, 472) reliée au système de soupape (300) selon une liaison de transmission de gaz contient un élément de stockage d'énergie.

11. Moteur (100) selon la revendication 10, **caractérisé en ce que** l'élément de stockage d'énergie est un ressort.

12. Moteur (100) selon les revendications 6 à 11, **caractérisé en ce que** le système de travail (200) est relié, selon une liaison de transmission de gaz, au système de soupape (300) par l'intermédiaire d'une première conduite de système de travail (275), et le système de commande (400) est relié, selon une liaison de transmission de gaz, au système de soupape (300) par l'intermédiaire d'une première conduite de système de commande (475), le quotient de l'ouverture de section transversale de la première conduite de système de travail (275) par rapport à l'ouverture de section transversale de la première conduite de système de commande (475) étant supérieur ou égal au quotient de la somme des volumes de la première section de cylindre de travail (271) et de la seconde section de cylindre de travail (272) par rapport à la somme des volumes de la première section de cylindre de commande (471) et de la seconde section de cylindre de commande (472).

13. Moteur (100) selon l'une quelconque des revendications précédentes, **caractérisé en ce que** le système de travail (200) présente un élément de travail (210).

14. Moteur (100) selon la revendication 13, **caractérisé en ce que** l'élément de travail (210) est relié au piston de travail (260) par liaison de force et/ou de forme, ou **en ce que** l'élément de travail (210) et le piston de travail (260) sont réalisés d'un seul tenant.

15. Moteur (100) selon la revendication 13 ou 14, **caractérisé en ce que** l'élément de travail (210) peut être équipé d'un moyen d'usinage, en particulier pour le sciage, le fraisage, le brossage, le perçage ou la pulvérisation de matière osseuse.

16. Moteur (100) selon l'une quelconque des revendications précédentes, **caractérisé en ce que** le moteur (100) est fabriqué en matière plastique, en métal ou à partir d'une combinaison de matière plastique et de métal.

17. Moteur (100) selon l'une quelconque des revendications précédentes, **caractérisé en ce que** le piston de travail (260) et le piston de commande (460) sont accouplés de sorte qu'une fréquence de commande du piston de commande (460) ne subit pratiquement pas l'influence d'une fréquence de travail du piston de travail (260).

18. Moteur (100) selon l'une quelconque des revendications précédentes, **caractérisé en ce que** le piston de travail (260) présente un diamètre compris entre 10 et 40 mm, de préférence entre 15 et 35 mm, plus préférablement entre 20 et 30 mm.

19. Dispositif médical, en particulier pour le sciage, le fraisage, le brossage, le perçage ou la pulvérisation de matière osseuse, **caractérisé en ce que** le dispositif médical présente un moteur (100) selon l'une quelconque des revendications précédentes.

20. Dispositif médical selon la revendication 19, **caractérisé en ce que** le dispositif médical présente une poignée, le système de soupape (300) étant disposé à l'intérieur de la poignée.
